# EUROPÄISCHE PATENTSCHRIFT

(11) **EP 3 768 153 B1**
(45) Veröffentlichungstag und Bekanntmachung des Hinweises auf die Patenterteilung: **14.08.2024**
(21) Anmeldenummer: 19712999.2
(22) Anmeldetag: 22.03.2019
(51) Int. Cl.: A61B 5/00, G01N 21/64, G02B 21/00

(54) **MULTIMODALES BILDGEBUNGSSYSTEM UND VERFAHREN ZUR NICHT-INVASIVEN UNTERSUCHUNG EINES UNTERSUCHUNGSOBJEKTS**
MULTIMODAL IMAGING SYSTEM AND METHOD FOR NON-INVASIVE EXAMINATION OF AN OBJECT
SYSTÈME D'IMAGERIE MULTIMODALE ET PROCÉDÉ D'EXAMEN NON INVASIF D'UN OBJET D'EXAMEN

(30) Priorität: 23.03.2018 EP 18163646
(43) Veröffentlichungstag der Anmeldung: 27.01.2021
(73) Patentinhaber: JenLab GmbH, 12559 Berlin (DE)
(72) Erfinder: KÖNIG, Karsten, Berlin (DE); WEINIGEL, Martin, Görlitz (DE)
(74) Vertreter: Simmons & Simmons LLP (Munich)
(86) Internationale Anmeldenummer: PCT/EP2019/057182
(87) Internationale Veröffentlichungsnummer: WO 2019/180187

(56) Entgegenhaltungen:
- WO-A1-2017/156182
- DE-A1- 10 065 146
- DE-A1- 102010 047 578
- US-A1- 2006 129 036
- US-A1- 2014 023 993
- US-B2- 7 761 129

## Beschreibung

Die Erfindung betrifft ein multimodales Bildgebungssystem und ein Verfahren zur nicht-invasiven Untersuchung eines Untersuchungsobjekts.

### Stand der Technik

US 5,034,613 A, DE 20 117 294 U1, DE 20 306 122 U1, und DE 10 2010 047 578 A1 offenbaren starre und flexible Systeme zur Multiphotonen-Bildgebung mit gepulster Laserstrahlung im nahen Infrarotbereich. Beispielsweise werden mit der Multiphotonen-Bildgebung hochaufgelöste mikroskopische Bildfelder mit Kantenlängen im Bereich deutlich unter 1 mm generiert.

In DE 10 2009 029 831 A1 wird vorgeschlagen, eine so genannte fliegende Optik zu verwenden, wobei eine Optikeinheit zur Fokussierung einer Anregungsstrahlung und zur Anregung einer Sekundärstrahlung nicht fest angeordnet ist, sondern insgesamt bewegt wird, um unterschiedliche Orte eines zu untersuchenden Objekts zeitlich nacheinander anzuregen. Die örtliche Anregung erfolgt dabei nicht durch Strahlablenkung unter Verwendung von Dreh- und Kippspiegeln, sondern durch eine ein- oder mehrdimensionale Bewegung der Optikeinheit im Ganzen. Nachteilig daran ist die geringe Reaktionszeit der motorisierten Optikeinheit und, daraus folgend, eine geringe Bildaufnahmerate.

Aus DE 10 2011 115 944 A1 ist ein flexibles nichtlineares Laserscanning-Mikroskop zur nicht-invasiven dreidimensionalen Untersuchung eines Untersuchungsobjekts bekannt, welches eine Strahlungsquelle aufweist, die einen Anregungsstrahl zur Auslösung einer von Atomen und Molekülen emittierten Sekundärstrahlung erzeugt, und weiterhin eine flexible Übertragungsoptik zur Übertragung der Strahlung zu einem Messkopf mit einer Fokussieroptik, mit der die Strahlung in das Messobjekt fokussiert wird. Der Messkopf ist dabei frei im Raum schwenkbar, drehbar und flexibel positionierbar mit der Strahlungsquelle verbunden, so dass Mikroskopie unter beliebigen Raumwinkeln ausführbar ist. Weitere Bildgebungssysteme und Verfahren entsprechend des Stands der Technik werden zum Beispiel in DE10065146A1, US2014/0023993A1, US2006/0129036A1, US7761129B2 und WO2017/156182A1 beschrieben.

Um eine multivalente Untersuchung am menschlichen Körper durch den flexibel positionierbaren Messkopf zu ermöglichen, ist in DE 10 2011 115 944 A1 vorgesehen, dass sich der Messkopf mechanisch durch einen flexiblen in beliebiger Stellung arretierbaren Stützarm auf einem mobilen Grundgerät abgestützt, der die Übertragungsoptik mitführt. In dem Grundgerät sind die Strahlungsquelle zur Erzeugung gepulster Anregungsstrahlung, sowie Optikeinheiten zur Ausrichtung der Anregungsstrahlung auf mindestens eine Übertragungsoptik zum Messkopf vorgesehen. Um die gewünschte Genauigkeit bei der flexiblen Strahlübertragung durch den Spiegelgelenkarm zu erreichen, ist eine Richtungsstabilisierung für die Laserstrahlung vorgesehen mit einem Detektor, einer Steuereinheit und einem motorisierten Kippspiegel, wobei abhängig von einer detektierten Abweichung in einer konjugierten Zielebene innerhalb des Messkopfes mittels der Steuereinheit eine Korrektur der Strahlrichtung des Laserstrahls vorgenommen wird. In DE 10 2011 115 944 A1 ist die Überlagerung des mikroskopischen Bildfelds der Fokussieroptik und der Messposition mit Unsicherheiten verbunden. Da die Fokussieroptik einen Durchmesser von ca. 30 mm und einen Arbeitsabstand von ca. 0,2 mm zum Untersuchungsobjekt aufweist, ist das mikroskopische Bildfeld vollständig durch die Fokussieroptik überdeckt.

Die relative Position zwischen der Fokussieroptik und dem Untersuchungsobjekt ist darüber hinaus abhängig von der Reproduzierbarkeit der mechanischen Kopplung zwischen dem Messkopf und dem Untersuchungsobjekt bzw. einer Adapterplatte zur Kontaktierung des Untersuchungsobjekts.

Demgegenüber stellt sich die vorliegende Erfindung die Aufgabe, den Anwender bei der Positionierung des Messkopfes gegenüber dem zu untersuchenden Untersuchungsobjekt zu unterstützen. Die Präzision der Positionierung, mit der eine gewünschte Zielposition (ROI, engl. *region of interest*) des zu untersuchenden Untersuchungsobjektes hochaufgelöst bildgebend dargestellt werden kann, ist insbesondere von der Präzision der mechanischen Positionierbarkeit der Fokussieroptik des multimodalen Bildgebungssystems abhängig.

### Offenbarung der Erfindung

Bei dem erfindungsgemäßen multimodalen Bildgebungssystem handelt es sich um ein Bildgebungssystem nach Anspruch 1.

Unter "multimodal" wird im Folgenden verstanden, dass zwei, drei oder vier Systeme bereitgestellt sind, die zumindest teilweise miteinander integriert sind. Die Systeme können sich einzelne Komponenten miteinander teilen, beispielsweise wenn ein oder mehrere Mess- oder Bildgebungssysteme dieselbe Strahlungsquelle, Übertragungsoptik, Fokussieroptik oder dasselbe Detektorsystem verwenden. Vorteilhaft wird eine kompakte Anordnung vorgeschlagen, bei der einzelne Komponenten von mehreren Systemen zur gleichen Zeit oder nacheinander verwendet werden. Beispielsweise können die Systeme nacheinander oder gleichzeitig verschiedene Bildgebungsmodi ermöglichen.

Insbesondere ist zumindest die Fokussieroptik, mittels welcher die nahe infrarote Femtosekundenlaserstrahlung auf das Untersuchungsobjekt richtbar ist, und die konfokale Detektionseinrichtung in dem Messkopf integriert bereitgestellt und sind damit frei im Raum schwenkbar, drehbar und flexibel positionierbar. Darüber hinaus ist vorgesehen, dass auch der Laserkopf der Strahlungsquelle mit in dem Messkopf integriert bereitgestellt ist. Beispielsweise kann hierzu die Quelle des Anregungsstrahls abgesehen von der Energieversorgung und dem Treiber vollständig im Messkopf integriert sein. Vorteilhaft benötigen die Laserstrahlen somit keine Ein- oder Auskopplung in einen Gelenkarm des multimodalen Bildgebungssystems, so dass die Positionierung des Messkopfes keinen Einfluss z. B. auf die Übertragungsqualität haben, wodurch die Fehleranfälligkeit des Systems sinkt.

Der Messkopf weist darüber hinaus auch ein Minimum an Übertragungsoptik auf, welche beispielsweise im einfachsten Fall eine Scaneinheit, eine Scanlinse, eine Tubuslinse, einen dichroitischen Strahlteiler und die Fokussieroptik umfassen kann. Das resultierende Bildfeld eines solchen Laserscanning-Systems kann beispielsweise 350 µm x 350 µm sein. Der Messkopf kann darüber hinaus auch einen x-y-Translationstisch mit einem Positionierbereich von z. B. 5 mm x 5 mm umfassen. Vorteilhaft wird mit der Integration diverser Komponenten in den Messkopf ein sehr kompaktes System vorgeschlagen, das hochaufgelöste Detailaufnahmen von biologischem und nichtbiologischem Gewebe bereitstellt. Das System ist insgesamt kleiner, leichter und ergonomischer als Vorgängersysteme.

Unter "nicht-invasiv" wird im Folgenden verstanden, dass die Bildgebungssysteme mechanisch nicht in das Untersuchungsobjekt eindringen. Nicht-invasive Verfahren zur Bildgebung sind invasiven Verfahren typischerweise vorzuziehen, um die Risiken von etwaiger Kontamination oder Zerstörung des Untersuchungsobjekts zu vermeiden. Das vorgeschlagene multimodale Bildgebungssystem zur nicht-invasiven Untersuchung erlaubt Untersuchungen beispielsweise an lebenden Untersuchungsobjekten, zum Beispiel an Pflanzen, Tieren und Menschen, insbesondere beispielsweise Haut- oder Augenuntersuchungen an Menschen durchzuführen. Insbesondere können nicht-invasiv auch Tiefeninformationen aus epidermalen und/oder dermalen Hautschichten mit mikroskopischer Auflösung bereitgestellt werden, beispielsweise zur Feststellung von Hautkrebs.

Unter "frei im Raum schwenkbar, drehbar und flexibel positionierbar" wird verstanden, dass der Messkopf z. B. mechanisch durch einen flexiblen in beliebigen Stellungen arretierbaren Stützarm an einem Grundgerät abgestützt bereitgestellt wird. Der Messkopf und das Grundgerät führen dabei jeweils Teile des multimodalen Bildgebungssystems mit. Das Grundgerät selbst kann mobil sein. In dem Grundgerät können beispielsweise ein Teil der Strahlungsquelle, nämlich zum Beispiel die Energieversorgung der Strahlungsquelle vorgesehen sein und auch Computersysteme, Benutzerschnittstellen und Teile von Detektorsystemen zur Messung der emittierten Sekundärstrahlung oder des weiteren Systems zur Bereitstellung von Übersichtsbildern von dem Untersuchungsobjekt.

Unter "hochaufgelösten Detailbildern" wird verstanden, dass das vorgestellte multimodale Bildgebungssystem optische Mikroskopie und Tomographie mit horizontalen Auflösungen im Submikrometerbereich und Scanbereichen von einigen 100 Mikrometern in drei Dimensionen ermöglicht. Die Bildfeldgröße des Detailbildes ist beispielsweise 350 µm x 350 µm horizontal und 200 µm vertikal.

Die "Messposition" des Untersuchungsobjekts wird im Folgenden auch als ROI bezeichnet.

Das Multiphotonen-Bildgebungssystem kann insbesondere ein Multiphotonen-Fluoreszenzmikroskop, auch Mehrphotonen-Fluoreszenzmikroskop sein. Bei einem Multiphotonen-Fluoreszenzmikroskop handelt es sich um ein spezielles Lichtmikroskop aus der Gruppe der Laserscanning-Mikroskope. Die emittierte Sekundärstrahlung kann insbesondere Fluoreszenzstrahlung sein, welche entsteht, wenn das Untersuchungsobjekt Photonen absorbiert und zu einem späteren Zeitpunkt ein anderes Photon wieder abgibt. Durch das absorbierte Photon wird ein Elektron eines Atoms oder Moleküls des Untersuchungsobjekts auf ein höheres Energieniveau angehoben und speichert die Energie des anregenden Photons zwischen. Nach einer für das entsprechende Atom oder Molekül charakteristischen Zeit fällt das Elektron wieder zurück auf sein niedrigeres Energieniveau und sendet dabei ein neues Photon aus. Während bei der herkömmlichen Ein-Photonen-Fluoreszenzmikroskopie lediglich ein Photon benötigt wird, um das Elektron auf das höhere Energieniveau anzuregen, sind es bei der Mehrphotonen-Fluoreszenzmikroskopie mehrere Photonen. Das verbreitetste Verfahren ist dabei die Zwei-Photonenfluoreszenzmikroskopie (TPFM, engl. *two-photon fluorescence microscopy*)*,* wobei die Anregung des Elektrons genau durch zwei Photonen erfolgt, die in der Summe die gleiche Energie aufweisen, die das Elektron benötigt, um auf das höhere Niveau angehoben zu werden. Voraussetzung für die Anregung ist, dass die zwei Photonen gleichzeitig eintreffen, da typischerweise kein stabiles Zwischenenergieniveau des anzuregenden Elektrons existiert. Für weiterführende Hinweise wird auf die Veröffentlichung von Denk et al., "Two-Photon Laser Scanning Fluorescence Microscopy", Science, Vol. 248, S. 73-76 verwiesen.

Abgesehen von der TPF (eng. *two-photon fluorescence*) können vom Multiphotonen-Bildgebungssystem auch frequenzverdoppelte Signale (SHG, engl. *second harmonic generation*) und frequenzverdreifachte Signale (THG, engl. *third harmonic generation*) der Anregungsstrahlung gemessen werden. Ein Detektorsystem des Multiphotonen-Bildgebungssystems kann daher vorteilhaft einen oder mehrere Detektoren zur TPF-, SHG- und THG-Bildgebung aufweisen, so dass ein flexibles Diagnosesystem für fluoreszierende und nichtfluoreszierende Substanzen insbesondere in lebender Materie vorhanden ist. Insbesondere ist auch eine gleichzeitige und ortsgleiche Bildgebung aus TPF-, SHG- und THG-Signalen möglich.

Alternativ oder zusätzlich dazu kann das Detektorsystem des Multiphotonen-Bildgebungssystems eine Vorrichtung zur zeitkorrelierten Einzelphotonenzählung (TCSPC, engl. *time-correlated single photon counting*) aufweisen, insbesondere zur Erfassung der charakteristischen Zwei-Photonenfluoreszenzlebensdauern unterschiedlicher endogener und exogener Fluorophore.

"Femtosekundenlaserstrahlung" bezeichnet in der vorliegenden Offenbarung insbesondere gepulste Laserstrahlung mit Pulsdauern von z. B. wenigen hundert Femtosekunden oder bevorzugt von weniger als 100 fs in Abgrenzung zu Piko- und Attosekundenpulsen. Der Begriff Femtosekundenlaserstrahlung grenzt sich auch zu CW-Lasern (engl. *continuous wave*) ab. Um hohe Photonendichten in der Messposition des Untersuchungsobjekts zu erzeugen, umfasst der Anregungsstrahl ultrakurz gepulste Laserstrahlung mit Pulsdauern im Femtosekundenbereich.

Als Laser kommen beispielsweise Laser mit Modenkopplung in Betracht. Insbesondere kann der Laser Laserdioden als Pumpquelle verwenden. Als Lasereinheit kann beispielsweise ein Erbiumfaserlaser infrage kommen, der einen Anregungsstrahl mit einer Wellenlänge von z. B. 1560 nm erzeugt, wobei die Anregungsstrahlung durch einen Frequenzverdoppler auf entsprechend 780 nm halbiert wird. Zur Anregung der Atome und Moleküle des Untersuchungsobjekts wird die frequenzverdoppelte Strahlung verwendet.

Alternativ können Halbleiterlaser vorgesehen sein. Zwar ist die Repetitionsrate der modengekoppelten Laser für die zeitaufgelöste Fluoreszenzlasermikroskopie derzeit noch etwas hoch, aber für einfache Intensitätsmessungen kann ein solcher Laser bereits berücksichtigt werden.

Ein weiteres Beispiel eines Femtosekundenlaserstrahlung emittierenden Lasers ist ein Titan-Saphir-Laser mit einer Laserpulsbreite von weniger als 200 fs oder weniger als 100 fs, einer Wiederholfrequenz von 80 MHz oder 90 MHz, einer Leistung von 0 - 1,5 W und einem Wellenlängenbereich von 710 - 920 nm.

Die Fokussieroptik des Multiphotonen-Bildgebungssystems umfasst beispielsweise ein Linsensystem, mit welchem der Anregungsstrahl auf das Untersuchungsobjekt fokussierbar und defokussierbar ist.

Die Fokussieroptik weist beispielsweise eine numerische Apertur von 0,8 bis 1,4, bevorzugt von 1 bis 1,4, insbesondere beispielsweise von 1,3 auf, welche sich als vorteilhaft für die Multiphotonen-Bildgebung, insbesondere für die Zweiphotonen-Bildgebung herausgestellt hat.

Die oben genannte konfokale Detektionseinrichtung ist vorteilhaft zumindest teilweise, bevorzugt vollständig mit in den Messkopf integriert und eingerichtet, um ein aus dem Fokusvolumen innerhalb des Untersuchungsobjekts remittiertes, z. B. reflektiertes Signal des Anregungsstrahls naher infraroter Femtosekundenlaserstrahlung zu empfangen. Verwendet werden kann das remittierte Signal insbesondere zur Bildgebung.

Aufgrund der starken instantanen Reflexionssignale sind im Vergleich zur Autofluoreszenz-basierten Multiphotonen-Bildgebung vorteilhafterweise deutlich kürzere Pixelverweilzeiten erforderlich, um eine hinreichende morphologische Bildgebung des Untersuchungsobjekts zu ermöglichen. Diese Eigenschaft der konfokalen Detektionseinrichtung kann vorteilhaft für eine "schnelle" Übersichtsbildgebung des Untersuchungsobjekts genutzt werden. Übersichtsbilder können dabei durch das schachbrettförmige Zusammenfügen einer Vielzahl angrenzender mikroskopischer Bildfelder generiert werden, beispielsweise eine Vielzahl angrenzender Bildfelder von beispielsweise 350 x 350 µm². Die Bilder werden nacheinander aufgenommen und schachbrettartig zu einem Übersichtsbild zusammengefügt. Die konfokale Detektionseinrichtung verwendet hierzu bevorzugt eine hohe numerische Apertur der Fokussieroptik, z. B. von 0,8 bis 1,4, bevorzugt von 1 bis 1,3. Die Bildfeldgrößen der Multiphotonen-Bildgebung und die der konfokalen Detektionseinrichtung sind nahezu identisch.

Vorteilhaft wird damit zur räumlichen Orientierung, d.h. zur Unterstützung des Anwenders bei der Positionierung des Messkopfes gegenüber dem zu untersuchenden Untersuchungsobjekt also der Einsatz eines komplementären bildgebenden Verfahrens und Systems mit einem aus einer Vielzahl kleinerer Bildfelder zusammengesetzten "großen" Übersichtsbildfeld vorgeschlagen, dessen Koordinatensystem z. B. eine räumliche Korrelation der Multiphotonen-Bildgebung zur gewünschten Messposition des Untersuchungsobjekts erlaubt. Während konventionelle stationäre Multiphotonen-Mikroskope aufgrund ihres starren Aufbaus einen begrenzten Anwendungsbereich bieten, eignet sich das vorgestellte multimodale Bildgebungssystem außerdem zur multivalenten Untersuchung am menschlichen Körper.

Gemäß einer bevorzugten Ausführungsform sind zumindest Teile des Detektorsystems des Bildgebungssystems mit in dem Messkopf integriert bereitgestellt.

Die integriert bereitgestellten Teile des Detektorsystems sind beispielsweise Sekundärelektronenvervielfacher (PMT, engl. *photomultiplier tube*)*,* CCD-Felder, CMOS-Felder, oder ähnliche Detektoren, die die empfangene emittierte Sekundärstrahlung des Untersuchungsobjekts in elektronische Datensignale umwandeln können. Vorteilhaft wird mit der Integration von Teilen des Detektorsystems in den Messkopf ein sehr kompaktes System vorgeschlagen, das die Aufnahme großflächiger Übersichtsbildfelder und hochaufgelöster Detailaufnahmen von biologischem und nichtbiologischem Gewebe bereitstellt.

Nach einer vorteilhaften Ausführungsform umfasst das multimodale Bildgebungssystem zumindest ein weiteres System zur Bereitstellung von Übersichtsbildern von dem Untersuchungsobjekt, wobei das weitere System zumindest teilweise mit in den Messkopf integriert ist, und wobei das weitere System eine CCD-Kamera oder CMOS-Kamera und/oder einen optischen Kohärenztomographen umfasst. Vorteilhaft ist das weitere System somit ebenfalls frei im Raum schwenkbar, drehbar und flexibel positionierbar.

Unter "Übersichtsbildern" werden Bilder mit beispielsweise mehreren Millimetern Kantenlänge verstanden. Der Erfassungsbereich des weiteren Systems zur Aufnahme von Übersichtsbildern kann daher z. B. mindestens einen Quadratmillimeter, beispielsweise mindestens 2 x 2 mm², 5 x 5 mm² oder 10 x 10 mm² groß ausgebildet sein. Im Folgenden werden durch den Begriff "Übersichtsbild" sowohl zweidimensionale als auch dreidimensionale Übersichtsbilder verstanden. Beispielsweise ist bei der optischen Kohärenztomographie ein dreidimensionales Bild mit großem Bildfeld möglich. Gemäß einer Ausführungsform ist das weitere System zur Bereitstellung von Übersichtsbildern von dem Untersuchungsobjekt Kamera-basiert und umfasst eine CCD-Kamera oder eine CMOS-Kamera.

Alternativ oder zusätzlich hierzu kann das weitere System einen optischen Kohärenztomographen aufweisen. Die optische Kohärenztomographie (OCT, engl. *optical coherence tomography*) ermöglicht vorteilhaft 2D- oder 3D-Übersichtsbilder inklusive topographischer Informationen von dem Untersuchungsobjekt mit hohem diagnostischen Potential. Der optische Kohärenztomograph kann aber auch eindimensional betrieben werden.

Die Kopplung von OCT-basierten Übersichtsaufnahmen mit der hochauflösenden Multiphotonen-Bildgebung ist durch unterschiedliche Anforderungen an die jeweilige Fokussieroptik erschwert, denn bei der Multiphotonen-Bildgebung wird typischerweise eine hohe numerische Apertur der Fokussieroptik benötigt, wohingegen bei der OCT nur eine kleine numerische Apertur vorteilhaft ist. Die vorliegende Erfindung bietet zu diesen divergierenden Anforderungen eine Lösung, bei welcher zwei verschiedene optische Systeme zum Einsatz kommen. Zunächst erfolgt die OCT-basierte Übersichtsbildgebung und im Anschluss die hochauflösende Multiphotonen-Bildgebung an der entsprechenden Zielposition (ROI). Sowohl das System zur OCT- als auch das zur Multiphotonen-Bildgebung sind in einem gemeinsamen Messkopf integriert und werden nacheinander, ohne eine mechanische Entkopplung des Untersuchungsobjektes vom Messkopf angewandt. Es ist offensichtlich, dass die Zielposition (ROI) bei einer Multiphotonen-Fokussieroptik mit einem Durchmesser von z. B. 30 mm und einem Arbeitsabstand von ca. 0,2 mm vollständig durch die Fokussieroptik überdeckt wird und keine optische Zugänglichkeit für die großflächige OCT-Bildgebung bietet. Zwischen Multiphotonen-Fokussieroptik und Untersuchungsobjekt wird daher zunächst in Z-Richtung mit einem Linearaktuator ein größerer Abstand von z. B. 10 mm eingestellt. Eine seitliche und in z. B. 45° gekippte Anordnung des OCT-Bildgebungssystems bietet die notwendige optische Zugänglichkeit zur Zielposition für die OCT-basierte Übersichtsbildgebung, ohne dass das Bildfeld des OCT-Bildgebungssystems durch die äußeren Abmessungen der Multiphotonen-Fokussieroptik beschnitten wird. Die mechanische Kopplung beider Bildgebungssysteme und der Bezug innerhalb des gemeinsamen Koordinatensystems erlaubt die räumliche Korrelation zwischen beiden Bildgebungsmodalitäten. Das OCT-Bildgebungssystem kann hierbei durch ein scannendes Element, beispielsweise ein x-y-Galvanometerscanner, ein optisches System mit einer weiteren Fokussieroptik geringer numerischer Apertur, einen interferometrischen Messaufbau, ein Spektrometer, eine spektral breitbandige Beleuchtung und eine Auswerteeinheit realisiert werden. Das scannende Element erzeugt hierbei die 2D-Auslenkung des für die optische Kohärenzmessung notwendigen Messstrahls.

Bei einer vorteilhaften Ausführungsform ist das weitere System dazu eingerichtet, um die nahe infrarote Femtosekundenlaserstrahlung als Beleuchtungsstrahlung zu verwenden. Beim Aufnehmen des Übersichtsbildes kann also die Beleuchtung insbesondere durch die Femtosekunden-Laserstrahlungsquelle erfolgen.

Gemäß einer Ausführungsform ist die CCD-Kamera oder CMOS-Kamera seitlich an der Front des Messkopfes unter Einhalten der Scheimpflug'schen Regel angeordnet.

Die Anordnung von Kamera, Sensor, abbildender Linse und Objekt erfolgt vorteilhaft nach der so genannten Scheimpflug'schen Regel, bei der die Objekt-, Bild- und Linsenebene eine gemeinsame Schnittachse aufweisen. Die Winkel zwischen den Ebenen werden so ausgelegt, dass die Abbildungsbedingung in allen Objekt- und konjugierten Bildpunkten erfüllt ist. Aus der seitlichen Bildgebung mit nichtparallelen Objekt- und Bildebenen resultieren unterschiedliche Abbildungsmaßstäbe innerhalb des Bildfeldes. Diese Bildverzerrungen werden durch eine computergestützte perspektivische Entzerrung korrigiert.

Alternativ hierzu kann eine Kamera-basierte Bildgebung vorgesehen sein, wobei die CCD-Kamera oder CMOS-Kamera die Fokussieroptik, insbesondere die Multiphotonen-Fokussieroptik, als abbildendes Element verwendet.

Durch den Einsatz der Fokussieroptik als bildgebender Bestandteil der Kameraanordnung wird vorteilhaft eine sehr kompakte Baugröße des Systems erreicht. Die Kamera kann vorteilhaft im Inneren des Messkopfs positioniert sein. Die Abbildungsbedingung wird dabei durch eine Objektweite erfüllt, die deutlich größer ist als der Arbeitsabstand der Fokussieroptik zum Untersuchungsobjekt, beispielsweise größer als das 10fache, das 20fache, das 30fache oder größer als das 50fache, insbesondere zwischen dem 20fachen und dem 70fachen, z. B. das 50fache. Die optimale Objektweite ist durch die gewünschte Objektgröße, d. h. die Kantenlänge des Übersichtsbildes, definiert. Durch die große Objektweite und die hohe numerische Apertur der Fokussieroptik ist eine großflächige Ausleuchtung des Gewebes mit dem Anregungsstrahl linear polarisierter Femtosekundenlaserstrahlung gegeben. Typische Objektweiten bei einer Fokussieroptik mit einer Brennweite von ca. 4,1 mm stellen 2 bis 10 mm dar. Es ist bekannt, dass die Fokussieroptik in dieser Anordnung optisch unkorrigiert ist und entsprechende (mono-)chromatische Aberrationen berücksichtigt werden müssen. Insbesondere werden Bildverzerrungen induziert, die durch eine computergestützte perspektivische Entzerrung korrigiert werden.

Für den Kamerasensor kann die Kopplung des Beleuchtungs- und des Abbildungsstrahlengangs beispielsweise durch einen descanned positionierten Polarisationsstrahlteiler erfolgen. Vom Gewebe reflektierte unpolarisierte, durch die Fokussieroptik erfasste Anregungsstrahlung wird durch den descanned positionierten Polarisationsstrahlteiler partiell reflektiert und über ein Linsensystem zum Kamerasensor geführt. Chromatische Aberrationen können an dieser Stelle durch eine schmalbandige Beleuchtung gering gehalten werden. Schmalbandig kann dabei beispielsweise eine Bandbreite von weniger als 50 nm, bevorzugt weniger als 20 nm, weiter bevorzugt weniger als 10 nm bezeichnen. Alternativ kann der Polarisationsstrahlteiler durch einen Strahlteiler mit Intensitätsaufteilung ersetzt werden.

Es kann vorgesehen sein, dass die Kamera zur Übersichtsbildgebung manuell eingewechselt wird. Hierzu wird zunächst die Kamera mit der Adapterplatte gekoppelt. Im Anschluss an die Übersichtsbildaufnahme wird die Kamera entfernt und der Messkopf mit der Einrichtung zur hochaufgelösten mikroskopischen Bildgebung aufgesetzt.

Beispielsweise kann als weiteres System eine Kamera vorgesehen sein, die als eine weitere Strahlungsquelle für die Kamera-basierte Bildgebung eine Beleuchtungseinheit zur Erzeugung von visuellem oder nahinfrarotem Licht umfasst.

Für den Fall, dass diese Kamera "non-descanned" angeordnet ist, ist es zweckmäßig, wenn die Multiphotonen-Bildgebung und auch die Bildgebung durch die konfokale Detektionseinrichtung durch die weitere Strahlungsquelle unbeeinträchtigt sind, da sich der Abbildungsstrahlengang teilweise mit dem Anregungsstrahlengang überschneidet. Für die weitere Strahlungsquelle wird daher eine weitere Strahlungsquelle vorgeschlagen mit einem Spektralbereich, welcher sich nicht mit dem Spektralbereich der Detektions- oder Anregungsstrahlung der Multiphotonen-Bildgebung oder der Bildgebung durch die konfokale Detektionseinrichtung überschneidet. Diese Bedingung wird beispielsweise erfüllt durch einen Spektralbereich oberhalb von 950 nm und durch die Verwendung eines Strahlteilers im Strahlengang.

Bei einer vorteilhaften Ausführungsform ist zur Reduktion einer Amplitude des Zentralreflexes die konfokale Detektionseinrichtung derart angeordnet, dass sie ein partiell transmittiertes Signal eines der Strahlungsquelle vorgelagerten Umlenkspiegels abgreift. Es kann vorgesehen sein, dass das Signal daraufhin in einen zweiten Lichtleiter geleitet wird.

Der Umlenkspiegel kann dabei eine Reflektivität von mehr als 80%, bevorzugt von mehr als 90%, weiter bevorzugt mehr als 95% besitzen. Das transmittierte Signal wird nach Passage eines Analysators und einer Linse entweder direkt auf einen im Messkopf angeordneten Konfokaldetektor mit vorgelagerter Pinhole gegeben oder erst in einen Lichtleiter eingekoppelt und dann auf einen extern angeordneten Konfokaldetektor gegeben. Der Lichtleiter übernimmt dabei die Funktion der Pinhole.

Vorteilhaft werden durch die gekreuzte Polarisationsdetektion mit dem Analysator die bei der konfokalen Detektionseinrichtung aufgrund von Oberflächenreflexen der Beleuchtungsstrahlung an den verschiedenen optischen Komponenten auftretenden Bildartefakte in Form eines so genannten Zentralreflexes verringert.

Alternativ ist zur Reduktion einer Amplitude des Zentralreflexes ein Polarisationsstrahlteiler zur Trennung der linear polarisierten Anregungsstrahlung und reflektierter unpolarisierter Sekundärstrahlung des Untersuchungsobjekts vorgesehen, wobei die konfokale Detektionseinrichtung derart angeordnet ist, dass sie die remittierte unpolarisierte Sekundärstrahlung des Untersuchungsobjekts empfängt. Bei dieser Anordnung wird das linear polarisierte Signal der Strahlungsquelle den Polarisationsstrahlteiler durchlaufen und durch die Fokussieroptik im Untersuchungsobjekt fokussiert. Im Fokusvolumen wird die Strahlung reflektiert und unter Mehrfachstreuung partiell in Rückwärtsrichtung durch die Fokussieroptik erfasst. Der Polarisationszustand wird bei der Mehrfachstreuung an komplexen Gewebestrukturen des Untersuchungsobjekts gestört. Das in Rückwärtsrichtung über die Scanner geleitete (descanned) Signal wird partiell durch den Polarisationsstrahlteiler ausgekoppelt und konfokal erfasst, d.h. nach Passage einer Linse entweder direkt auf einen im Messkopf angeordneten Konfokaldetektor mit vorgelagerter Pinhole gegeben oder erst in einen Lichtleiter eingekoppelt, dessen Kerndurchmesser dem Durchmesser von z. B. einem Airy-Scheibchen entspricht, und dann auf einen extern angeordneten Konfokaldetektor gegeben. Durch diese ebenfalls gekreuzte Detektion wird ein Teil der Oberflächenreflexe unterbunden und die Amplitude des Zentralreflexes reduziert.

Bei einer vorteilhaften Ausführungsform weist die konfokale Detektionseinrichtung eine Vorrichtung zur zeitaufgelösten Signalverarbeitung des Remissionssignals des Anregungsstrahls naher infraroter Femtosekundenlaserstrahlung auf.

Über die zeitaufgelöste Detektion der konfokal detektierten Signale kann eine weitere Reduzierung und idealerweise vollständige Kompensation des Zentralreflexes erreicht werden. Dabei bietet sich entweder das oben erwähnte und für die Fluoreszenzlebensdauermessung bestimmte System zur zeitaufgelösten Einzelphotonenzählung (TCSPC) an oder eine einfache und kostengünstige Tor-Schaltung (GPC, engl. *gated photon counting*)*.* Mit einer TTSbegrenzten Zeitauflösung der Detektoren von zum Beispiel 200 ps resultiert eine Wegauflösung von 60 mm in Luft (TTS, engl. *transient time spread*)*.* Bei einer Zeitauflösung von 20 ps beträgt diese Strecke in Luft bereits 6 mm. Hierbei können die Oberflächenreflexe der meisten Optiken im Anregungsstrahlengang eliminiert werden.

Eine weitere vorteilhafte Ausführungsform ist die Bereitstellung einer Autofokuseinheit innerhalb des multimodalen Bildgebungssystems. Diese Einheit kann durch ein Messsystem, eine Auswerteeinheit und einen Linearaktuator, welcher auch als z-Aktuator bezeichnet sein kann, umgesetzt werden und zur Positionierung bzw. Nachführung der Fokussieroptik in Bezug zu dem sich axial bewegenden Untersuchungsobjekt dienen.

Mittels der Autofokuseinheit kann insbesondere die Kopplung zwischen dem Untersuchungsobjekt und dem Bildgebungssystem stabilisiert und damit lebendes Gewebe wesentlich besser untersucht werden. Die Fixierung des Gewebes in lateraler Richtung ist oftmals sehr gut durch die Kombination von doppelseitigem Klebeband, einem Deckglas und einem magnetischen Adapterring stabilisiert. In axialer Richtung wird dann durch die vorgeschlagene Autofokuseinheit die Objektebene und die Fokusposition stabilisiert, welche sich insbesondere durch Puls, Atmung oder sonstige Relativbewegungen des Untersuchungsobjekts ändern. Insbesondere kann durch die Autofokuseinheit die durch die Flexibilität der verwendeten Deckgläser induzierte Bildverzerrung (beispielsweise werden Deckgläser mit einer Dicke von 60 bis 180 µm verwendet, welche sich bei erhöhtem Anpressdruck des Untersuchungsobjekts durchbiegen) verringert oder idealerweise verhindert werden. Derart bedingte Bildverzerrungen werden im Folgenden auch als "Bildartefakte" bezeichnet. Gerade bei hochdynamischen Geweben, beispielsweise bei der Untersuchung von Hautarealen des Brustkorbes, können sich Atmungsartefakte störend auswirken. Die für die Bildauswertung wichtige Tiefeninformation geht ohne eine solche Nachführung durch einen Autofokus verloren, womit vertikale Schnittbilder, so genannte x-z- oder y-z-Scans, verzerrt werden und eine computergestützte Auswertung und eine 3D-Rekonstrution des Gewebes insbesondere bei En-face-Stapelaufnahmen schwer möglich ist.

Der z-Aktuator ist beispielsweise ein Schrittmotor oder kann auf Piezoelektronik basieren. Piezoaktuatoren erlauben eine schnelle Nachführung mit einer Grenzfrequenz größer 0,1 kHz und sind damit deutlich schneller als die meisten oben genannten Relativbewegungen lebender Objekte. Je nach Ausführungsform bieten diese einen Hub von 100 µm bis 500 µm.

Bei einer vorteilhaften Ausführungsform weist das multimodale Bildgebungssystem zur Bereitstellung einer Autofokus-Funktion eine Vorrichtung zur Bestimmung der Lage eines Deckglases und/oder des Untersuchungsobjekts auf.

Vorteilhaft kann das Sensorsignal für die Autofokuseinheit durch einen Oberflächenreflex des verwendeten flexiblen Deckglases bereitgestellt werden. Der Oberflächenreflex kann hierbei beispielsweise durch das integrierte System zur optischen Kohärenztomographie oder durch eine Lasertriangulation mit einem separaten System dynamisch ausgewertet werden.

Gemäß einer vorteilhaften Ausführungsform weist das multimodale Bildgebungssystem einen kollinear zur optischen Achse der Fokussieroptik ausgerichteten OC-Messstrahl, mit dessen Hilfe die Lage eines Deckglases und/oder des Untersuchungsobjekts bestimmbar ist, zur Bereitstellung der Autofokus-Funktion auf.

Die optische Kohärenzmessung (OC, engl. *optical coherence*) kann ohne Bildgebung vorteilhaft als ein Teil der Autofokuseinheit in das System integriert werden. Hierbei wird ein OC-Messstrahl über einen Strahlteiler mit der optischen Achse der Fokussieroptik überlagert. Um eine geringe effektive numerische Apertur und folglich hohe Rayleighlänge des OC-Messstrahls zu ermöglichen, wird die numerische Apertur der Fokussieroptik nicht vollständig ausgeleuchtet, sondern beispielsweise zu weniger als 30%, bevorzugt weniger als 20%, weiter bevorzugt weniger als 10%. Die optische Schicht des Strahlteilers ermöglicht die Transmission für die Beleuchtungsstrahlung der Multiphotonen-Bildgebung und der Bildgebung durch die konfokale Detektionseinrichtung im Spektralbereich von beispielsweise 710 bis 950 nm. Die zu detektierenden Sekundärsignale beispielsweise Fluoreszenz- und SHG-Signale im Spektralbereich von weniger als 710 nm und der Spektralbereich für den OC-Messstrahl werden reflektiert. Zur Integration können beispielsweise Superlumineszenzdioden (SLD) vorgesehen sein, welche beispielsweise eine Zentralwellenlänge um 1060 nm mit einer Bandbreite von 70 nm aufweisen, in Kombination mit faseroptischen Bauelementen und integrierten Spektrometern. Vorteilhaft bei dieser Anordnung ist, dass die übrigen hierin beschriebenen Bildgebungsmodalitäten nicht beeinträchtigt werden. Zudem wirkt sich durch die geringe Baugröße dieser Einheit und die Möglichkeit der distalen Anordnung der Superlumineszenzdioden und der Spektrometer beispielsweise in einem mobilen Rollwagengehäuse positiv auf das Gewicht und das Bauvolumen des Messkopfes aus.

Gemäß einer vorteilhaften Ausführungsform ist ein Drucksensor oder Kraftsensor zur Auffindung einer Oberfläche des Untersuchungsobjekts und/oder zur Kontrolle eines Anpressdrucks vorgesehen.

Die axiale Position des Objektes ist abhängig vom Anpressdruck. Vorteilhaft kann daher durch den Drucksensor das Sensorsignal für die Autofokuseinheit bereitgestellt werden. Der Drucksensor ist bevorzugt ein integrierter elektrischer Drucksensor und kann beispielsweise auf Dehnungsmessstreifentechnologie basieren.

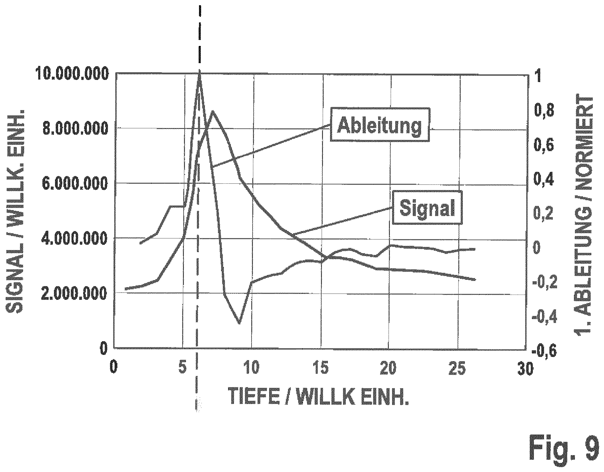

Durch den Drucksensor kann vorteilhaft auch bei der Initialisierung der hochauflösenden Bildgebung das Auffinden der Gewebeoberfläche erleichtert werden. Die Initialisierung der Bildaufnahme kann durch den Einsatz des Linearaktuators, der die Fokussieroptik axial in Bezug auf das Untersuchungsobjekt verschiebt, verbessert werden, indem beispielsweise kontinuierlich axial ausgelenkt wird und simultan die Auswertung der Multiphotonen-Fluoreszenzsignale in der ROI detektiert wird. Die Oberfläche des Untersuchungsobjekts kann dabei durch den Übergang von signalarmen Deckglas bzw. Immersionsfluid zur fluoreszierenden Probe definiert werden, vgl. hierzu Figur 9. Als Gewebeoberfläche wird beispielsweise der Wendepunkt von signalarmen zu signalreichen Bereichen definiert.

Durch Auswertung des Drucksensors kann der Anpressdruck vorteilhaft kontrolliert oder begrenzt werden. Insbesondere bei ophthalmologischen und bei dermatologischen Untersuchungen kann hierdurch verhindert werden, dass es zu einem zu großen Anpressdruck kommt, welcher die Blutzufuhr des Gewebes einschränken und dadurch den Metabolismus des Gewebes beeinträchtigen kann.

Gemäß einer vorteilhaften Ausführungsform ist eine Freigabesteuerung des Anregungsstrahls an eine Vorrichtung zur Feststellung der Präsenz des Untersuchungsobjekts im Messbereich gekoppelt.

Beispielsweise kann die Vorrichtung zur Feststellung der Präsenz des Untersuchungsobjekts durch den oben beschriebenen Drucksensor realisiert sein. Der Drucksensor dient damit vorteilhaft der Patientensicherheit. Registriert der Drucksensor das Untersuchungsobjekt, so kann der Anregungsstrahl freigegeben werden.

Alternativ oder zusätzlich hierzu kann vorgesehen sein, dass der Messstrahl zur optischen Kohärenztomographie zur Feststellung der Präsenz des Untersuchungsobjekts im Messbereich dient und somit vorteilhaft zur Patientensicherheit eingesetzt wird. Befindet sich beispielsweise Gewebe in der Objektebene, so wird dieses durch den Messstrahl zur optischen Kohärenzmessung registriert und hieraufhin der Anregungsstrahl freigegeben.

Ein Computersystem des multimodalen Bildgebungssystems umfasst beispielsweise Steuer- und Verarbeitungseinheiten, insbesondere bekannte Signalverarbeitung und/oder Bildverarbeitung. Darüber hinaus weist das multimodale Bildgebungssystem insbesondere beispielsweise Benutzerschnittstellen auf, wie zum Beispiel eine Maus, eine Tastatur, Anzeigen, einen Touchscreen oder Ähnliches. Über eine Benutzerschnittstelle kann beispielsweise das Übersichtsbild angezeigt werden und der Anwender kann eine Entscheidung über die Lage der Messposition treffen, welche detailliert zu untersuchen ist. Bei der Aufnahme des Detailbildes des Untersuchungsobjekts kann das Übersichtsbild überlagert dargestellt werden, so dass der Anwender erkennt, an welcher Position des Übersichtsbildes das Detailbild aufgenommen wird. Die Auswahl der Messposition kann über dieselbe Benutzerschnittstelle (Touchscreen) oder über eine weitere Benutzerschnittstelle (Maus, Tastatur o.ä.) stattfinden. Die Bilder können beispielsweise abgespeichert oder gedruckt werden und/oder anderen Modulen übergeben werden. Das Computersystem kann den Anwender dabei beispielsweise mit geeigneten Zoom- und Scrollfunktionen unterstützen. Weitere Werkzeuge können beispielsweise den Anwender bei der Berechnung von Abständen und Größen im Bild unterstützen.

Die Computereinheiten und Peripheriegeräte des Computersystems, wie Bildschirme, Ein- und Ausgabegeräte, usw., können sich vorteilhaft außerhalb des Messkopfes befinden, so dass dieser nicht unnötig schwer wird.

Bei einer weiteren vorteilhaften Ausführungsform ist das multimodale Bildgebungssystem batteriebetrieben.

Diese Ausführungsform ermöglicht vorteilhaft zeitweise einen Einsatz des kompakten Systems in beliebiger Umgebung unabhängig von dem Vorhandensein einer Energiequelle. Vorteilhaft kann das batteriebetriebene System für mehrere Stunden autark betrieben werden. Das batteriebetriebene System ist damit insbesondere ortsveränderlich, womit Untersuchungen direkt am Patientenbett oder "im Feld" möglich sind. Außerdem besteht vorteilhaft eine erhöhte Ausfallsicherheit gegenüber Datenverlust durch Stromausfall.

Bei einem erfindungsgemäßen Verfahren zur nicht-invasiven Untersuchung eines Untersuchungsobjekts unter Verwendung eines der multimodalen Bildgebungssysteme nach einem der Ansprüche 1 bis 9 werden die folgenden Schritte ausgeführt:
- Ausrichten der Fokussieroptik auf eine Messposition,
- Richten der nahen infraroten Femtosekundenlaserstrahlung der Strahlungsquelle auf die Messposition, und
- Messen der emittierten Sekundärstrahlung des Untersuchungsobjekts zur Erstellung eines hochaufgelösten Detailbildes des Untersuchungsobjekts an der Messposition nacheinander oder gleichzeitig durch das Multiphotonen-Bildgebungssystem und durch die konfokale Detektionseinrichtung.

Die zuvor in Bezug auf das System beschriebenen Merkmale sollen dabei auch für das Verfahren als offenbart gelten und umgekehrt.

Der Schritt des Messens der emittierten Sekundärstrahlung des Untersuchungsobjekts zur Erstellung des hochaufgelösten Detailbildes von dem Untersuchungsobjekt an der Messposition umfasst vorteilhaft eine kombinierte Messung durch mehrere Systeme. Insbesondere vorteilhaft stellt die Erfindung eine Kombination der Multiphotonen-Bildgebung mit hohen Bildraten der Bildgebung durch die konfokale Detektionseinrichtung bereit.

Aufgrund der starken instantanen Reflexionssignale sind im Vergleich zur Autofluoreszenz-basierten Multiphotonen-Bildgebung vorteilhafterweise deutlich kürzere Pixelverweilzeiten erforderlich, um eine hinreichende morphologische Bildgebung des Untersuchungsobjekts zu ermöglichen. Diese Eigenschaft der konfokalen Detektionseinrichtung kann vorteilhaft für eine "schnelle" Übersichtsbildgebung des Untersuchungsobjekts genutzt werden. Übersichtsbilder können dabei durch das schachbrettförmige Zusammenfügen einer Vielzahl angrenzender mikroskopischer Bildfelder generiert werden, beispielsweise eine Vielzahl angrenzender Bildfelder von beispielsweise 350 x 350 µm². Die Bilder werden nacheinander aufgenommen und schachbrettartig zu einem Übersichtsbild zusammengefügt.

Bei einer Ausführungsform des Verfahrens kann also eine Messung eines Übersichtsbilds erfolgen. Das Verfahren kann dabei die folgenden Schritte umfassen:
- Ausrichten des Messkopfes auf einen Übersichtsbereich des Untersuchungsobjekts,
- Aufnehmen eines Übersichtsbildes des Untersuchungsobjekts durch das konfokale Detektionssystem und/oder durch eine CCD-Kamera oder CMOS-Kamera und/oder einen optischen Kohärenztomographen und
- Auswählen einer Messposition in dem Übersichtsbereich zur Aufnahme des hochaufgelösten Detailbildes.

Beim Aufnehmen des Übersichtsbildes kann die Beleuchtung insbesondere durch die Femtosekunden-Laserstrahlungsquelle erfolgen.

Das hochaufgelöste Detailbild kann wie beschrieben gemessen werden durch
- Ausrichten der Fokussieroptik auf die Messposition,
- Richten der nahen infraroten Femtosekundenlaserstrahlung der Strahlungsquelle auf die Messposition, und
- Messen der emittierten Sekundärstrahlung des Untersuchungsobjekts zur Erstellung eines hochaufgelösten Detailbildes des Untersuchungsobjekts an der Messposition durch das Multiphotonen-Bildgebungssystem und/oder durch die konfokale Detektionseinrichtung.

Bei einer Weiterbildung des Verfahrens kann zunächst ein Übersichtsbild durch die Kamera erfolgen, hierauf eine Bildgebung durch die konfokale Detektionseinrichtung und schließlich eine Messung durch die hochauflösende Multiphotonen-Bildgebung. Auch hier kann die Beleuchtung stets durch die Femtosekunden-Laserstrahlungsquelle erfolgen.

Bei einer vorteilhaften Ausführungsform ist vorgesehen, dass axiale Bewegungsartefakte des Untersuchungsobjekts durch eine Autofokusfunktion korrigiert werden, indem eine laufende mechanische Nachjustierung des Abstands der Fokussieroptik zum Untersuchungsobjekt erfolgt.

Weiter kann vorteilhaft vorgesehen sein, dass Messsignale mit dem Ziel zur Auffindung der Oberfläche des Untersuchungsobjekts ausgewertet werden, insbesondere zur Bereitstellung einer Autofokus-Funktion.

Nach einer vorteilhaften Ausführungsform wird beim Aufnehmen des Übersichtsbildes des Untersuchungsobjekts ein größerer Abstand zwischen der Fokussieroptik und dem Untersuchungsobjekt eingestellt als beim Aufnehmen des Detailbildes des Untersuchungsobjekts. Die Einstellung des Abstands kann insbesondere durch den Linearaktuator erfolgen, der die Fokussieroptik axial in Bezug auf das Untersuchungsobjekt verschiebt.

Alternativ hierzu kann das Übersichtsbild von der CCD-Kamera oder CMOS-Kamera und/oder von dem optischen Kohärenztomographen nicht kollinear zur optischen Achse der Fokussieroptik als ein Schrägbild aufgenommen werden. Nach einer vorteilhaften Ausführungsform erfolgt eine Freigabe des Anregungsstrahls, wenn das Untersuchungsobjekt im Messbereich präsent ist. Hierdurch kann z. B. die Patientensicherheit gewährleistet werden.

Zur Auffindung der Position der Gewebeoberfläche in Bezug auf die Objektebene des hochauflösenden Bildgebungssystems können in einer Ausführungsform Multiphotonen-Fluoreszenzsignale ausgelesen werden, welche von dem Kreatin in der Hautoberfläche erzeugt werden. Eine präzisere Lokalisierung der Hautoberfläche lässt sich durch die Bestimmung des Wendepunktes zwischen signalarmen Bereichen des Glases bzw. der Wasserimmersionsschicht und der kreatinreichen Hautoberfläche ermitteln.

Nach einem weiteren Aspekt der Erfindung wird eines der multimodalen Bildgebungssysteme nach einem der Ansprüche 1 bis 9 zur Untersuchung lebender Materie des Untersuchungsobjekts verwendet. Das Verfahren eignet sich also insbesondere zur in-vivo-Untersuchung von Menschen, Tieren oder Pflanzen. Durch die vorgeschlagene Anordnung mit frei positionierbarem flexiblem Messkopf kann eine Untersuchung von beliebig ausgerichteten Objektoberflächen nicht-invasiv erfolgen, insbesondere z. B. auch eine Vielzahl von Messungen an verschiedenen Teilen eines beispielsweise ausgedehnten Untersuchungsobjekts.

Insbesondere kann eine Anwendung an Proben vorgesehen sein, die mit biologischen Fluoreszenzmarkern wie zum Beispiel GFP markiert wurden.

Allgemein kann die Verteilung typischer endogener Fluorophore wie Collagen, Elastin, Flavine, Keratin, NAD (P)H und/oder Melanin innerhalb des Gewebes bestimmt, und daraus z. B. Rückschlüsse auf krankhafte Gewebeneubildung, wie z. B. Krebs gebildet werden.

Durch Frequenzverdopplung der Anregungsstrahlung können beispielsweise aber auch Collagenfasern oder Myosin sehr gut sichtbar gemacht werden.

Das System eignet sich daher auch bei der diagnostischen Unterstützung eines Arztes, der insbesondere In-vivo-Detailbilder der menschlichen Haut erhält und hieraus ergänzende Unterstützung zu seinen Diagnoseentscheidungen erhalten kann.

### Kurze Beschreibung der Figuren

In den Figuren sind mögliche Ausführungsbeispiele der Erfindung dargestellt.

Es zeigen:
- Figur 1: eine schematische Darstellung eines multimodalen Bildgebungssystems gemäß einer Ausführungsform der Erfindung,
- Figur 2: eine schematische Darstellung eines multimodalen Bildgebungssystems gemäß einer weiteren Ausführungsform der Erfindung,
- Figur 3: zwei schematische Darstellungen eines Untersuchungsobjekts bei der Untersuchung durch ein multimodales Bildgebungssystem gemäß einer Ausführungsform der Erfindung zu verschiedenen Zeitpunkten,
- Figur 4: eine schematische Darstellung eines Untersuchungsobjekts bei der Untersuchung durch ein multimodales Bildgebungssystem gemäß einer Ausführungsform der Erfindung,
- Figur 5: eine schematische Darstellung eines Untersuchungsobjekts bei der Untersuchung durch ein multimodales Bildgebungssystem gemäß einer weiteren Ausführungsform der Erfindung,
- Figur 6: eine schematische Darstellung eines Untersuchungsobjekts bei der Untersuchung durch ein multimodales Bildgebungssystem gemäß einer weiteren Ausführungsform der Erfindung,
- Figur 7: eine schematische Darstellung eines Teils eines Messkopfes eines multimodalen Bildgebungssystems gemäß einer weiteren Ausführungsform der Erfindung,
- Figur 8: eine perspektivische Darstellung eines Untersuchungsobjekts bei der Untersuchung durch ein multimodales Bildgebungssystem gemäß einer Ausführungsform der Erfindung, und
- Figur 9: eine Darstellung einer Signalstärke über eine Eindringtiefe.

### Ausführungsformen der Erfindung

Die im Folgenden mit Bezug zu den Figuren beschriebenen Ausführungsbeispiele sind für den Gegenstand der Erfindung nicht als einschränkend aufzufassen. Die Figuren stellen den Gegenstand nur schematisch dar.

In den Figuren sind gleiche oder ähnliche Komponenten mit gleichen Bezugszeichen versehen, wobei in der Beschreibung in Einzelfällen auf eine wiederholte Nennung dieser Komponenten verzichtet wird.

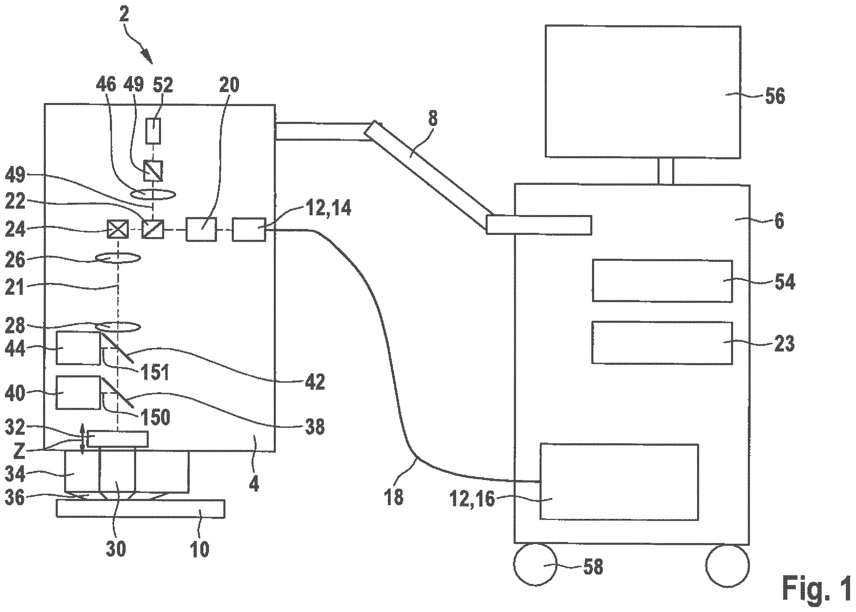

Figur 1 zeigt eine schematische Darstellung eines multimodalen Bildgebungssystems 2 gemäß einer ersten Ausführungsform der Erfindung.

Das multimodale Bildgebungssystem 2 umfasst einen Messkopf 4, der frei im Raum schwenkbar, drehbar und flexibel positionierbar ist, so dass eine Untersuchung eines Untersuchungsobjektes 10 unter beliebigen Raumwinkeln ausführbar ist.

Zu dem Zweck der freien Positionierbarkeit ist der Messkopf 4 mittels eines Gelenkarms 8 an einem mobilen Grundgerät 6 befestigt.

Das multimodale Bildgebungssystem 2 umfasst eine Strahlungsquelle 12, die einen im Messkopf angeordneten Laserkopf 14 und einen im mobilen Grundgerät 6 angeordneten Lasertreiber 16 umfasst. Der Laserkopf 14 und der Lasertreiber 16 sind über einen ersten Lichtleiter 18, z. B. eine optische Faser miteinander verbunden. In einigen Ausführungsformen (nicht dargestellt) kann der erste Lichtleiter 18 im Gelenkarm 8 integriert sein.

Der Lasertreiber 16 umfasst beispielsweise die Energieversorgung des Lasers und Pumpdioden. Der Laserkopf 14 umfasst eine SHG-Einheit, welche die vom Lasertreiber 16 emittierte Strahlung zur Erzeugung eines linear polarisierten Anregungsstrahls 21 frequenzverdoppelt.

Der Anregungsstrahl 21 passiert eine Leistungseinstellungseinheit 20, einen Polarisationsstrahlteiler 22 und wird über eine Scaneinheit 24 und über zwei Linsen 26, 28 auf eine Fokussieroptik 30 gelenkt, welche den Anregungsstrahl 21 auf das Untersuchungsobjekt 10 fokussiert. Die Scaneinheit 24 erlaubt eine Winkelauslenkung der Anregungsstrahlung in zwei Ebenen, welche mittels Fokussieroptik 30 in eine zweidimensionale Translationsbewegung des Anregungsvolumens überführt wird.

Die Fokussieroptik 30 ist mittels eines Linearaktuators 32 in einer durch Pfeile angedeuteten z-Richtung einstellbar. Hierzu umfasst der Linearaktuator 32 einen Motor. Im dargestellten Ausführungsbeispiel entspricht die z-Richtung der optischen Achse der Fokussiereinheit 30.

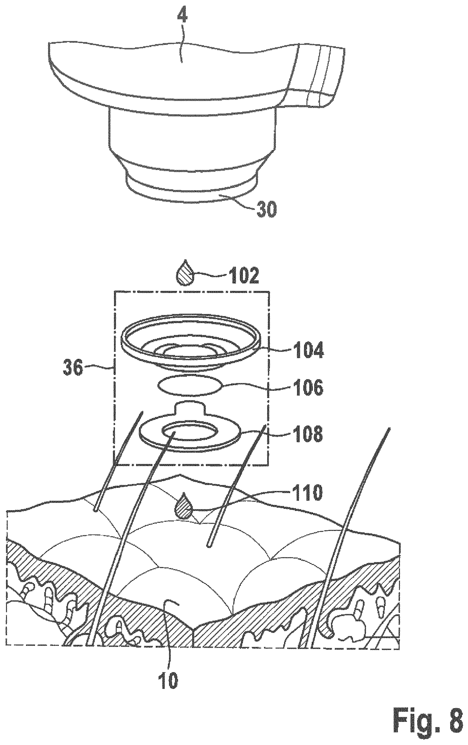

Der gesamte Messkopf 4 ist durch den Gelenkarm 8 an einer beliebigen Position im Raum arretiert. Zwischen der Fokussieroptik 30 und dem Untersuchungsobjekt 10 ist eine Adapterplatte 36 vorgesehen, welche in Kontakt mit dem Untersuchungsobjekt 10 tritt. Die Adapterplatte 36 wird mit Bezug zu Figur 8 näher beschrieben. Ein x-y-Translationstisch 34 ist eingerichtet, um die Adapterplatte 36 und das an die Adapterplatte 36 gekoppelte Untersuchungsobjekt 10 lateral in Bezug auf den Messkopf 4 zu bewegen. Der x-y-Translationstisch 34 umfasst hierzu entsprechende motorbetriebene Aktuatoren.

Der Anregungsstrahl 21 wechselwirkt mit dem Untersuchungsobjekt 10. Das Untersuchungsobjekt 10 emittiert hieraufhin eine Sekundärstrahlung, beispielsweise Reflexionsstrahlung, Fluoreszenzstrahlung, insbesondere TPF, SHG, oder THG, welche von der Fokussieroptik 30 aufgenommen wird. Im dargestellten Ausführungsbeispiel sind zwischen der zweiten Linse 28 und der Fokussieroptik 30 zwei dichroitische Strahlteiler 38, 42 vorgesehen, welche die TPF-, SHG- oder THG-Signale auf einen ersten und einen zweiten Detektor 40, 44 senden. Beispielsweise kann der erste Detektor 40 zum Erfassen der SHG-Signale und der zweite Detektor 44 zum Erfassen der TPF-Signale ausgebildet sein. In einigen Ausführungsformen (nicht dargestellt) kann nur einer der dichroitischen Strahlteiler 38, 42 zwischen der Linse 28 und der Fokussieroptik 30 positioniert werden. Bei dieser Ausführungsform kann die spektrale Trennung der Signale in einem der angekoppelten ersten oder zweiten Strahlengänge 150, 151 mit weiteren Strahlteilern erfolgen.

Im dargestellten Ausführungsbeispiel ist eine konfokale Detektionseinrichtung in das multimodale Bildgebungssystem 2 integriert. Die konfokale Detektionseinrichtung umfasst den Polarisationsstrahlteiler 22, eine Linse 46, eine Pinhole 49 und einen Konfokalstrahlungsdetektor 52. Die konfokale Detektionseinrichtung nimmt einen Teil der vom Anregungsstrahl 21 am Untersuchungsobjekt 10 ausgelösten unpolarisierten Reflexionsstrahlung auf. Die Reflexionsstrahlung wird hierzu nach dessen Passage der Scaneinheit 24 sogenannt "descanned" abgegriffen. Das Signal wird nach der Passage der Scaneinheit 24 am Polarisationsstrahlteiler 22 reflektiert und über die Linse 46 und die Pinhole 49 auf den Konfokalstrahlungsdetektor 52 geführt, welcher auch als CLSM-Detektor bezeichnet wird. Der Konfokalstrahlungsdetektor 52 befindet sich im dargestellten Ausführungsbeispiel im Messkopf 4. Als Beleuchtung für die konfokale Detektionseinrichtung dient die Strahlungsquelle 12.

Durch das oben beschriebene Abgreifen des Signals, dessen Polarisationszustand von dem des Anregungsstrahls abweicht, wird vorteilhaft eine Reduzierung der Amplitude des Zentralreflexes erreicht. Eine weitere Reduzierung bzw. vollständige Kompensation kann in einigen Ausführungsformen über eine zeitaufgelöste Detektion des Konfokalsignals erfolgen, wobei hierzu der Konfokalstrahlungsdetektor 52 entsprechende Vorrichtungen aufweist, beispielsweise eine Tor-Schaltung.

In einer nicht dargestellten alternativen Ausführungsform zur Erfassung der konfokal detektierten Fluoreszenz kann der Polarisationsstrahlteiler 22 durch einen dichroitischen Spiegel ersetzt, die dichroitischen Strahlteiler 38, 42 entfernt und der Detektionsstrahlengang 160 mit einem Fluoreszenz-Bandpass erweitert werden.

Das mobile Grundgerät 6 umfasst neben dem Lasertreiber 16 typischerweise auch Signaleingänge (nicht dargestellt) für die von den Detektoren 40, 44 und vom Konfokalstrahlungsdetektor 52 empfangenen Signale oder Bilder zur Multiphotonen- und CLSM-Bildgebung.

Das mobile Grundgerät 6 umfasst außerdem eine Steuereinheit 54, mittels welcher beispielsweise Benutzereingaben verarbeitet werden können, die einzelnen Komponenten wie beispielsweise die Energiequellen gesteuert werden können, und mittels welcher auch ein vom Anwender oder von einer Triggereinheit initiierter Wechsel zwischen Übersichtsbild und Detailbild des Untersuchungsobjekts 10 verarbeitet werden kann. Als Teil der Schnittstelle zum Anwender weist das mobile Grundgerät eine Anzeige 56 auf, beispielsweise ein Touchscreen oder ein Monitor.

Außerdem sind Räder 58 angedeutet, um zu zeigen, dass das multimodale Bildgebungssystem 2 mobil ist. Als Energieversorgung ist für das multimodale Bildgebungssystem 2 eine Batterieeinheit 23 vorgesehen.

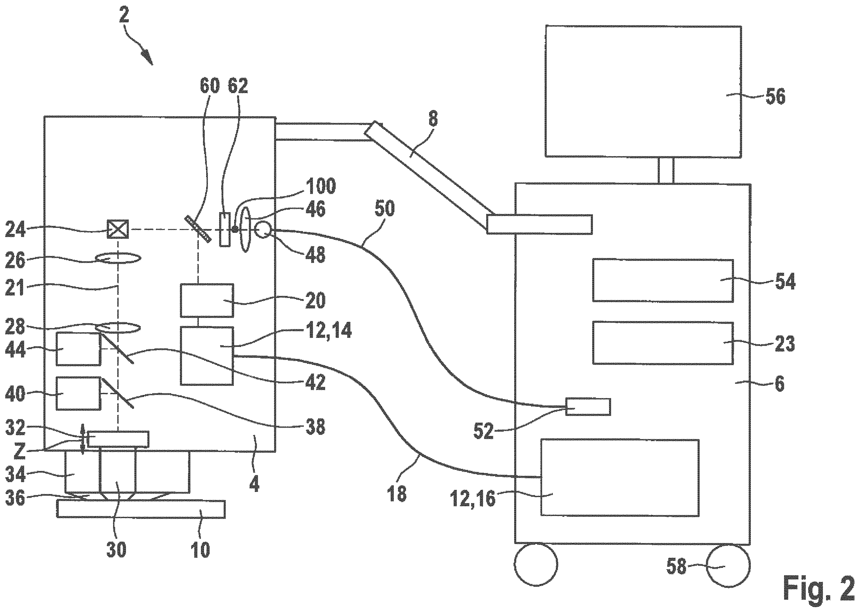

Figur 2 zeigt eine alternative Ausführungsform des multimodalen Bildgebungssystems 2. Das in Figur 2 dargestellte multimodale Bildgebungssystem 2 umfasst ebenfalls eine konfokale Detektionseinrichtung.

Der von der Strahlungsquelle 12 erzeugte Anregungsstrahl 21 wird bei dieser Ausführungsform zunächst über einen Umlenkspiegel 60 mit hoher Reflektivität, beispielsweise mehr als 90% oder mehr als 95%, aber weniger als 100% umgelenkt. Der weitere Strahlengang des Anregungsstrahls 21 ist wie mit Bezug zu Figur 1 beschrieben. Das von der Fokussieroptik 30 am Untersuchungsobjekt 10 erfasste Sekundärsignal wird über die Scaneinheit 24 als descanned-Signal zurückgeführt.

Die konfokale Detektionseinrichtung nimmt das Sekundärsignal als partiell transmittiertes Signal des Umlenkspiegels 60 ab. Das partiell transmittierte Signal wird hierzu nach der Passage des Umlenkspiegels 60 über einen Analysator 62 geführt, der so ausgerichtet ist, dass die Polarisationszustände der Anregungsstrahlung 21 und die transmittierte Detektionsstrahlung 100 orthogonal zueinander polarisiert sind (gekreuzte Polarisation). Die transmittierte Detektionsstrahlung 100 wird in dem hier dargestellten Ausführungsbeispiel über die Linse 46 an einer Einkopplungsstelle 48, die sich in dem Messkopf 4 befindet, in einen zweiten Lichtleiter 50 eingekoppelt, dessen Kerndurchmesser dem Durchmesser von z. B. einem Airy-scheibchen entspricht, und dem Konfokalstrahlungsdetektor 52 zugeführt. Der Konfokalstrahlungsdetektor 52 befindet sich im dargestellten Ausführungsbeispiel im Grundgerät 6. Auch dieses Ausführungsbeispiel mit Detektion in gekreuzter Polarisationsanordnung ermöglicht eine effektive Unterdrückung des Zentralreflexes.

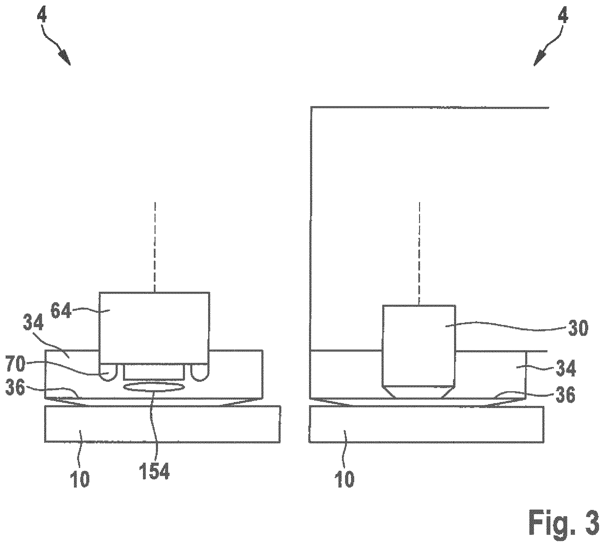

Figur 3 zeigt ein Untersuchungsobjekt 10 bei der Untersuchung durch ein multimodalen Bildgebungssystem 2 gemäß einer Ausführungsform der Erfindung zu verschiedenen Zeitpunkten, wobei jeweils ein Teil des Messkopfes 4 dargestellt ist, welcher z. B. eine Bildgebung wie mit Bezug zu Figur 1 oder 2 beschrieben ermöglicht.

In der linken Hälfte der Figur 3 ist die Kamera-basierte Übersichtsbildgebung dargestellt. Hierzu wird eine Kamera 64 mit der Adapterplatte 36 gekoppelt. Im dargestellten Ausführungsbeispiel besitzt die Kamera 64 eine weitere Strahlungsquelle 70 zur Erzeugung von sichtbarer Strahlung (VIS), z. B. Weißlicht und ein Kameraobjektiv 154.

Im Anschluss an die Übersichtsaufnahme wird die Kamera 64 entfernt und der Messkopf 4 mit der Einrichtung zur hochaufgelösten mikroskopischen Bildgebung bezüglich dem Untersuchungsobjekt 10 positioniert, wie rechts in Figur 2 dargestellt.

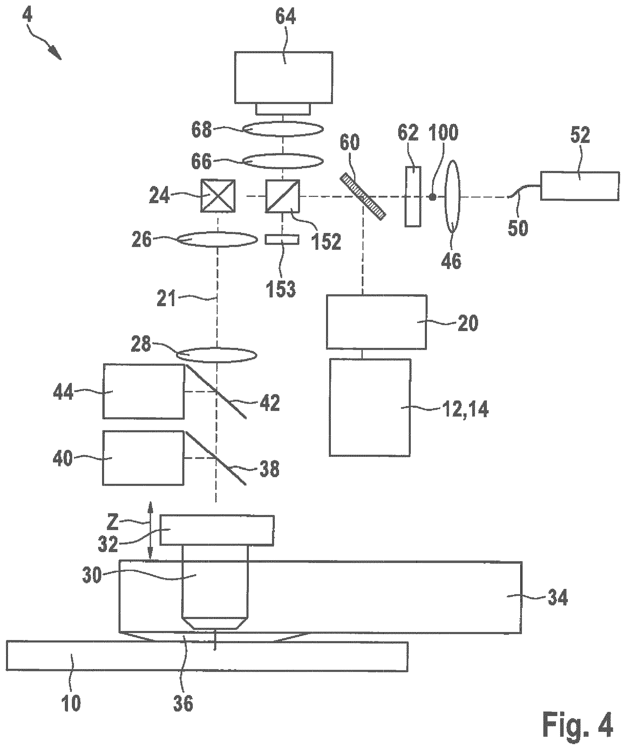

Figur 4 zeigt eine alternative Ausführungsform eines multimodalen Bildgebungssystems 2, welches die Multiphotonen-Bildgebung mit der konfokalen Detektionseinrichtung und zusätzlich einer Kamera-basierten Bildgebung koppelt.

Der Strahlengang der Multiphotonen-Bildgebung und der konfokalen Detektionseinrichtung kann wie mit Bezug zu Figur 2 beschrieben ausgestaltet sein. Die Kamera 64 ist vollständig im Messkopf 4 integriert. Die Kamera 64 verwendet die Fokussieroptik 30 als abbildendes Element und die Strahlungsquelle 12 als Beleuchtung. Die Abbildungsbedingung für die Kamera-basierte Bildgebung wird erfüllt, indem ein größerer Arbeitsabstand der Fokussieroptik 30 zum Untersuchungsobjekt 10 eingestellt wird. Beispielsweise ist hier eine Positionierung der Fokussieroptik 30 mittels des Linearaktuators 32 in einem Abstand von etwa 10 mm zum Untersuchungsobjekt 10 vorteilhaft. Durch die große Objektweite und die hohe numerische Apertur der Fokussieroptik 30 ist eine großflächige Ausleuchtung des Gewebes mit dem Anregungsstrahl 21 der Strahlungsquelle 12 gegeben. Das Signal passiert die Scaneinheit 24 und wird an einem Intensitäts-Strahlteiler 152 reflektiert. Der Intensitäts-Strahlteiler 152 hat zu diesem Zweck ein asymmetrisches Reflexions-/Transmissionsverhältnis, z. B. von weniger als 20/80, bevorzugt von weniger als 10/90, z. B. von 05/95. Ein Beamblock-Bauteil 153 ist eingefügt um einen Rückseitenreflex des Intensitäts-Strahlteilers 152 zu blocken. Das Signal wird hiernach über eine vierte und fünfte Linse 66, 68 der Kamera 64 zugeführt und dort durch den Kamerasensor ortsaufgelöst detektiert. Bei dieser Konfiguration befindet sich das Untersuchungsobjekt 10 in der Objektebene und der Kamerachip in der Bildebene des abbildenden optischen Systems. Die vierte und fünfte Linse 66 und 68 können auch durch ein deutlich komplexeres Linsensystem erweitert werden. Es versteht sich, dass der Messkopf 4 zur Übergabe des Kamerabildes an das mobile Grundgerät 6 entsprechende Schnittstellen aufweisen kann (nicht dargestellt).

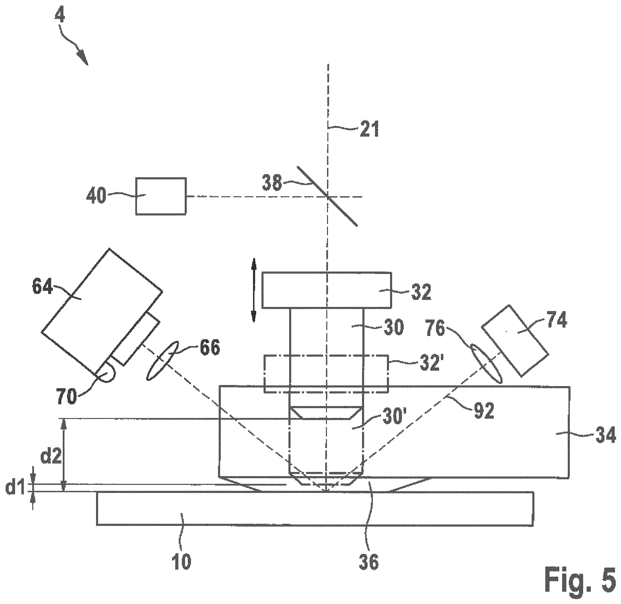

Figur 5 zeigt ein multimodales Bildgebungssystem 2 gemäß einer weiteren Ausführungsform. Das multimodale Bildgebungssystem 2 umfasst, z. B. zusätzlich zu den mit Bezug zu Fig. 1 oder 2 beschriebenen Bauteilen der Multiphotonen-Bildgebung und der konfokalen Detektionseinrichtung, eine seitlich am oder im Messkopf 4 integrierte Kamera 64, wobei die Anordnung des Kamerasensors 64, der abbildenden Linse 66 und des Untersuchungsobjektes 10 nach der Scheimpflug'schen Regel erfolgt, bei der die Objekt-, Bild- und Linsenebene eine gemeinsame Schnittachse aufweisen. Die Kamera 64 kann die Strahlungsquelle 12 als Beleuchtung verwenden. Alternativ oder zusätzlich hierzu ist die weitere Strahlungsquelle 70 zur Erzeugung VIS-Licht separat als eigenständige Einheit ebenfalls seitlich angeordnet am oder im Messkopf integriert.

Über den Linearaktuator 32 kann die Fokussieroptik 30 in z-Richtung verstellt werden, wobei in Figur 5 zwei verschiedene Einstellungen dargestellt sind. In einem ersten Abstand ist das multimodale Bildgebungssystem 2 zur Aufnahme von Detailbildern vom Untersuchungsobjekt 10 ausgerichtet und weist eine Objektweite d1 beispielsweise von 0,2 mm auf (durch gestrichene Bezugszeichen 30', 32' des Linearaktuators und der Fokussieroptik angedeutet). Bei einer zweiten Objektweite d2 von beispielsweise 10 mm (durch ungestrichene Bezugszeichen dargestellt) ist das multimodale Bildgebungssystem 2 zur Aufnahme der Kamera-basierten Übersichtsbilder eingerichtet. Im zweiten Fall bietet die seitliche und gekippte Anordnung die notwendige optische Zugänglichkeit für die Kamera-basierte Bildgebung, ohne dass der objektseitige Öffnungswinkel des Kamera-basierten Bildgebungssystems durch die äußeren Abmessungen der Fokussieroptik 30 beschnitten wird.

Das multimodale Bildgebungssystem 2 umfasst als ein weiteres System außerdem einen optischen Kohärenztomographen, welcher in Figur 5 durch ein optisches Kohärenzmesssystem 74 umfassend einen interferometrischen Messaufbau, Spektrometer, Beleuchtung und Auswerteeinheit, und eine weitere Fokussieroptik 76 angedeutet ist. Für die Integration der optischen Kohärenzmessung werden zur Beleuchtung beispielsweise Superlumineszenzdioden mit einer Zentralwellenlänge um 1060 nm und einer Bandbreite von 70 nm eingesetzt. Das optische Kohärenzmesssystem 74, die weitere Fokussieroptik 76 und ein OC-Messstrahl 92 des optischen Kohärenztomographen sind in einem Winkel von z. B. 30° bis 60°, bevorzugt von 40° bis 50°, insbesondere bevorzugt von 42° bis 48°, insbesondere beispielsweise 45° in Bezug auf die optische Achse des Anregungsstrahls 21 geneigt. Hierdurch kann der optische Kohärenztomograph bei der Einstellung des zweiten Abstands d2 eine Übersichtsaufnahme des Untersuchungsobjekts beispielsweise der Größenordnung 5 mm x 5 mm x 1 mm machen. Die seitliche und gekippte Anordnung bietet die notwendige optische Zugänglichkeit für den optischen Kohärenztomographen.

Durch zwei Galvanometerscanner (nicht dargestellt) kann eine zweidimensionale Auslenkung des OC-Messstrahls 92 erfolgen. Alternativ kann beispielsweise mit nur einem Galvanometerscanner eine eindimensionale Auslenkung des Messstrahls 92 erfolgen. Die 3D-Bildgebung kann im letzteren Fall aus einer Synchronisierung des resultierenden Linienscans mit der Vorschubbewegung der X- oder Y-Achse des motorisierten x-y-Translationstisches 34 erfolgen.

Selbstverständlich sind Ausführungsformen möglich, die lediglich das System mit der seitlich integrierten Kamera 64 oder lediglich den seitlich geneigt angeordneten optischen Kohärenztomographen aufweisen.

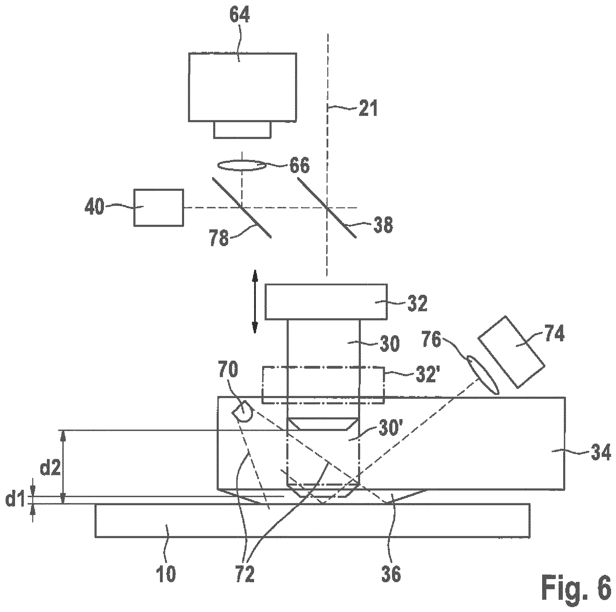

Figur 6 zeigt eine weitere Ausgestaltung eines multimodalen Bildgebungssystems 2 mit einer Multiphotonen-Bildgebung und einem optischen Kohärenztomographen wie in Bezug zu Figur 5 beschrieben. Als drittes System ist die Kamera 64 bei dieser Ausführungsform nicht seitlich angeordnet, sondern vollständig im Messkopf 4 integriert. Als abbildendes Element verwendet die Kamera 64 die Fokussieroptik 30. Bei dieser Konfiguration befindet sich das Untersuchungsobjekt 10 in der Objektebene und der Kamerachip in der Bildebene des abbildenden optischen Systems. Mit Bezugszeichen 78 ist ein dichroitischer Strahlteiler dargestellt. Als Beleuchtung wird eine an die Fokussiereinheit 30 oder an den Translationstisch 34 (dargestellt in Figur 6) gekoppelte separate weitere Strahlungsquelle 70 genutzt. Der Spektralbereich der Anregungsstrahlung 21 und der Spektralbereich der weiteren Strahlungsquelle 70 sind dabei disjunkt. Dargestellt ist auch ein Ausleuchtbereich 72 der weiteren Strahlungsquelle 70 zur Übersichtsbildgebung.

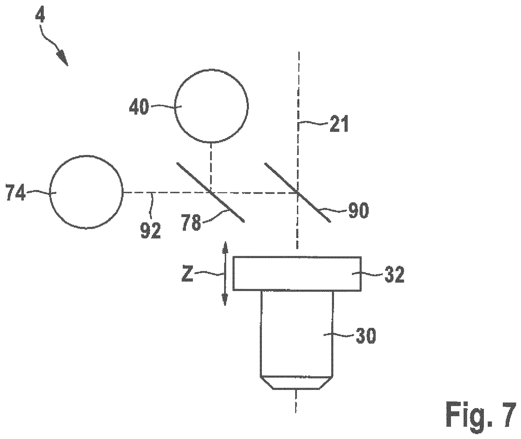

Figur 7 zeigt die Integration einer Autofokuseinheit durch Anwendung der optischen Kohärenz ohne Bildgebung. Hierbei wird der OC-Messstrahl 92 aus dem oben beschriebenen optischen Kohärenzmesssystem 74 über einen dritten dichroitischen Strahlteiler 90 in den Strahlengang der Anregungsstrahlung 21 eingekoppelt. Der OC-Messstrahl und die optische Achse von Fokussieroptik 30 sind dabei kollinear ausgerichtet. Die optische Schicht des dichroitischen Strahlteilers 90 ermöglicht beispielsweise die Transmission für die Beleuchtungsstrahlung mit dem Anregungsstrahl 21 im Spektralbereich von zum Beispiel 710 bis 950 nm. Die detektierten Sekundärsignale im Spektralbereich von unterhalb 710 nm sowie der Spektralbereich für den OC-Messstrahl 92 werden reflektiert. Um nur eine geringe effektive numerische Apertur und folglich hohe Rayleighlänge des OC-Messstrahls 92 zu erzielen, wird die Austrittspupille der Fokussieroptik 30 nicht vollständig ausgeleuchtet, sondern nur zu etwa 10% oder weniger.

Für die Integration der optischen Kohärenzmessung werden zur Beleuchtung beispielsweise Superlumineszenzdioden mit einer Zentralwellenlänge um 1060 nm und einer Bandbreite von 70 nm eingesetzt. Das Interferometer ist dabei ein Bestandteil des optisches Kohärenzmesssystems 74.

Figur 8 zeigt eine perspektivische Ansicht eines Messkopfes 4 mit einer Adapterplatte 36 und einem Untersuchungsobjekt 10, hier ein Teil menschlicher Haut. Die Adapterplatte 36 umfasst einen metallischen Kopplungsring 104, ein Deckglas 106 und einen Klebering 108 in dieser Reihenfolge. Zwischen der Fokussieroptik 30 des Messkopfs 4 und dem metallischen Kopplungsring 104 ist eine erste Immersionsflüssigkeit, beispielsweise ein Öl, vorgesehen, um den Wert der numerischen Apertur der Fokussieroptik 30 zu erhöhen. Zwischen dem Klebering 108 und dem Untersuchungsobjekt 10 ist eine zweite Immersionsflüssigkeit 110 vorgesehen, beispielsweise Wasser.

Figur 9 zeigt Messsignale zur Auffindung der Oberfläche des Untersuchungsobjekts 10. Die Oberfläche des Untersuchungsobjekts 10 kann durch den Übergang von signalarmen Deckglas bzw. Immersionsfluid zur fluoreszierenden Probe definiert werden. Als Gewebeoberfläche wird beispielsweise der Wendepunkt von signalarmen zu signalreichen Bereichen definiert.

### Bezugszeichenliste

| | | | |
|---|---|---|---|
| 2 | Multimodales Bildgebungssystem | 62 | Analysator |
| 4 | Messkopf | 64 | Kamera |
| 6 | mobiles Grundgerät | 68 | fünfte Linse |
| 8 | Gelenkarm | 70 | weitere Strahlungsquelle |
| 10 | Untersuchungsobjekt | 72 | Ausleuchtbereich |
| 12 | Strahlungsquelle | 74 | optisches Kohärenzmesssystem |
| 14 | Laserkopf | 76 | weitere Fokussieroptik |
| 16 | Lasertreiber | 78 | dritter dichroitischer Strahlteiler |
| 18 | erster Lichtleiter | 90 | vierter dichroitischer Strahlteiler |
| 20 | Leistungseinstellungseinheit | 92 | OC-Messstrahl |
| 21 | Anregungsstrahl | 100 | transmittierte Detektionsstrahlung |
| 22 | Polarisationsstrahlteiler | 102 | erste Immersionsflüssigkeit |
| 23 | Batterieeinheit | 104 | metallischer Kopplungsring |
| 24 | Scaneinheit | 106 | Deckglas |
| 26 | erste Linse | 108 | Klebering |
| 28 | zweite Linse | 110 | zweite Immersionsflüssigkeit |
| 30 | Fokussiereinheit | 150 | erster Strahlengang |
| 32 | Linearaktuator | 151 | zweiter Strahlengang |
| 34 | x-y-Translationstisch | 152 | Intensitäts-Strahlteiler |
| 36 | Adapterplatte | 153 | Beamblock-Bauteil |
| 38 | erster dichroitischer Strahlteiler | 154 | Kameraobjektiv |
| 40 | erster Detektor | 160 | Detektionsstrahlengang |
| 42 | zweiter dichroitischer Strahlteiler | | |
| 44 | zweiter Detektor | d1 | erste Objektweite |
| 46 | dritte Linse | d2 | zweite Objektweite |
| 48 | Einkopplungsstelle | | |
| 49 | Pinhole | | |
| 50 | zweiter Lichtleiter | | |
| 52 | Konfokalstrahlungsdetektor | | |
| 54 | Steuereinheit | | |
| 56 | Anzeige | | |
| 58 | Rad | | |
| 60 | Umlenkspiegel | | |

## Patentansprüche

1. Multimodales Bildgebungssystem (2) zur Bereitstellung eines Bilds eines Untersuchungsobjekts (10),
mit einem Multiphotonen-Bildgebungssystem zur Bereitstellung von hochaufgelösten Detailbildern von dem Untersuchungsobjekt (10), das eine Strahlungsquelle (12) mit einem Laserkopf (14) und einem Lasertreiber (16) aufweist, die einen Anregungsstrahl (21) naher infraroter Femtosekundenlaserstrahlung zur Auslösung einer von dem Untersuchungsobjekt (10) emittierten Sekundärstrahlung erzeugt, ein Detektorsystem (40, 44) zur TPF-, SHG- und THG-Bildgebung und/oder ein Detektorsystem zur zeitkorrelierten Einzelphotonenzählung, und eine Fokussieroptik (30), mittels welcher die Strahlung der Strahlungsquelle (12) auf eine Messposition des Untersuchungsobjekts (10) richtbar ist,
wobei die Fokussieroptik (30) und der Laserkopf (14) der Strahlungsquelle (12) in einem Messkopf (4) bereitgestellt sind, der frei im Raum schwenkbar, drehbar und flexibel positionierbar ist, sodass die Untersuchung des Untersuchungsobjekts (10) unter beliebigen Raumwinkeln ausführbar ist, und wobei der Laserkopf (14) eine SHG-Einheit zur Frequenzverdoppelung der von dem Lasertreiber (16) emittierten Strahlung zur Erzeugung des linear polarisierten Anregungslichtstrahls (21) umfasst,
und mit zumindest einer konfokalen Detektionseinrichtung welche zumindest teilweise mit in den Messkopf (4) integriert und eingerichtet ist, um ein vom Untersuchungsobjekt (10) remittiertes Signal des Anregungsstrahls (21) naher infraroter Femtosekundenlaserstrahlung zu empfangen.

2. Multimodales Bildgebungssystem (2) nach Anspruch 1, wobei zumindest Teile eines Detektorsystems des Bildgebungssystems mit in dem Messkopf (4) integriert bereitgestellt sind, insbesondere wobei das multimodale Bildgebungssystem (2) batteriebetrieben werden kann.

3. Multimodales Bildgebungssystem (2) nach einem der vorhergehenden Ansprüche mit zumindest einem weiteren System zur Bereitstellung von Übersichtsbildern von dem Untersuchungsobjekt (10), wobei das weitere System zumindest teilweise mit in den Messkopf (4) integriert ist und wobei das weitere System eine CCD-Kamera oder CMOS-Kamera (64) und/oder einen optischen Kohärenztomographen umfasst, insbesondere wobei das weitere System eingerichtet ist, um die nahe infrarote Femtosekundenlaserstrahlung als Beleuchtungsstrahlung zu verwenden, vorzugsweise
wobei die CCD-Kamera oder CMOS-Kamera (64) seitlich an der Front des Messkopfes (4) unter Einhalten der Scheimpflug'schen Regel angeordnet ist, oder
wobei die CCD-Kamera oder CMOS-Kamera (64) die Fokussieroptik (30) als abbildendes Element verwendet.

4. Multimodales Bildgebungssystem (2) nach einem der vorhergehenden Ansprüche, wobei zur Reduktion einer Amplitude des Zentralreflexes die konfokale Detektionseinrichtung derart angeordnet ist, dass sie ein partiell transmittiertes Signal eines der Strahlungsquelle (12) vorgelagerten Umlenkspiegels (60) abgreift, oder
wobei zur Reduktion einer Amplitude des Zentralreflexes ein Polarisationsstrahlteiler (22) zur Trennung von linear polarisierter Anregungsstrahlung und remittierter unpolarisierter Sekundärstrahlung des Untersuchungsobjekts (10) vorgesehen ist und die konfokale Detektionseinrichtung derart angeordnet ist, dass sie die remittierte unpolarisierte Sekundärstrahlung des Untersuchungsobjekts (10) empfängt.

5. Multimodales Bildgebungssystem (2) nach einem der vorhergehenden Ansprüche, wobei die konfokale Detektionseinrichtung eine Vorrichtung zur zeitaufgelösten Signalverarbeitung des Remissionssignals des Anregungsstrahls (21) naher infraroter Femtosekundenlaserstrahlung aufweist.

6. Multimodales Bildgebungssystem (2) nach einem der vorhergehenden Ansprüche, wobei das multimodale Bildgebungssystem (2) zur Bereitstellung einer Autofokus-Funktion eine Vorrichtung zur Bestimmung der Lage eines Deckglases (106) und/oder des Untersuchungsobjekts (10) aufweist.

7. Multimodales Bildgebungssystem (2) nach Anspruch 6, mit einem kollinear zur optischen Achse der Fokussieroptik (30) ausgerichteten OC-Messstrahl (92), mit dessen Hilfe die Lage eines Deckglases (106) und/oder des Untersuchungsobjekts (10) bestimmbar ist, zur Bereitstellung der Autofokus-Funktion.

8. Multimodales Bildgebungssystem (2) nach einem der vorhergehenden Ansprüche, mit einem Drucksensor zur Auffindung einer Oberfläche des Untersuchungsobjekts (10) und/oder zur Kontrolle eines Anpressdrucks.

9. Multimodales Bildgebungssystem (2) nach einem der vorhergehenden Ansprüche, wobei eine Freigabesteuerung des Anregungsstrahls (21) an eine Vorrichtung zur Feststellung der Präsenz des Untersuchungsobjekts (10) im Messbereich gekoppelt ist.

10. Verfahren zur nicht-invasiven Untersuchung eines Untersuchungsobjekts (10) unter Verwendung eines multimodalen Bildgebungssystems (2) nach einem der vorherigen Ansprüche, mit den folgenden Schritten:
Ausrichten der Fokussieroptik (30) auf eine Messposition,
Richten der nahen infraroten Femtosekundenlaserstrahlung der Strahlungsquelle (12) auf die Messposition, und
Messen der emittierten Sekundärstrahlung des Untersuchungsobjekts (10) zur Erstellung eines hochaufgelösten Detailbildes von dem Untersuchungsobjekt (10) an der Messposition nacheinander oder gleichzeitig durch das Multiphotonen-Bildgebungssystem und durch die konfokale Detektionseinrichtung.

11. Verfahren nach Anspruch 10, mit den folgenden weiteren Schritten:
Ausrichten des Messkopfes (4) auf einen Übersichtsbereich des Untersuchungsobjekts (10),
Aufnehmen eines Übersichtsbildes des Untersuchungsobjekts (10) durch das konfokale Detektionssystem und/oder durch eine CCD-Kamera oder CMOS-Kamera (64) und/oder einen optischen Kohärenztomographen (74) und
Auswählen einer Messposition in dem Übersichtsbereich zur Aufnahme des hochaufgelösten Detailbildes.

12. Verfahren nach Anspruch 10 oder 11, wobei axiale Bewegungsartefakte des Untersuchungsobjekts (10) durch eine Autofokus-Funktion korrigiert werden, indem eine laufende mechanische Nachjustierung des Abstands der Fokussieroptik (30) zum Untersuchungsobjekt (10) erfolgt.

13. Verfahren nach einem der Ansprüche 10 bis 12, wobei Messsignale mit dem Ziel zur Auffindung der Oberfläche des Untersuchungsobjekts (10) ausgewertet werden, insbesondere zur Bereitstellung einer Autofokus-Funktion.

14. Verfahren nach einem der Ansprüche 10 bis 13, wobei ein Übersichtsbild durch eine CCD-Kamera oder CMOS-Kamera (64) und/oder durch einen optischen Kohärenztomographen (74) aufgenommen wird und wobei
beim Aufnehmen des Übersichtsbildes des Untersuchungsobjekts (10) ein größerer Abstand zwischen der Fokussieroptik (30) und dem Untersuchungsobjekt (10) eingestellt wird als beim Aufnehmen des Detailbildes des Untersuchungsobjekts (10) und/oder
wobei das Übersichtsbild von der CCD-Kamera oder CMOS-Kamera (64) und/oder von dem optischen Kohärenztomographen (74) nicht kollinear zur optischen Achse der Fokussieroptik (30) als ein Schrägbild aufgenommen wird.

15. Verwendung eines multimodalen Bildgebungssystems (2) nach einem der Ansprüche 1 bis 9 zur Untersuchung lebender Materie des Untersuchungsobjekts (10).

## Claims

1. A multi-modal imaging system (2) for providing an image of an examination object (10),
comprising a multiphoton imaging system for providing high-resolution detailed images of the examination object (10), which imaging system comprises a radiation source (12) with a laser head (14) and a laser driver (16), which generates an excitation beam (21) of near-infrared femtosecond laser radiation for triggering secondary radiation emitted by the examination object (10), a detector system (40, 44) for TPF, SHG and THG imaging and/or a detector system for time-correlated single photon counting, and focussing optics (30), by means of which the radiation of the radiation source (12) can be directed to a measurement position of the examination object (10),
wherein the focusing optics (30) and the laser head (14) of the radiation source (12) are provided within a measuring head (4) which is pivotable, rotatable and flexibly positionable freely in space such that the examination of the examination object (10) performable under any desired solid angle, and wherein the laser head (14) comprises an SHG unit for frequency doubling of the radiation emitted by the laser driver (16) to generate the linearly polarised excitation beam (21),
and with at least one confocal detection device which is at least partially integrated into the measuring head (4) as well and is configured to receive a signal of the excitation beam (21) of near-infrared femtosecond laser radiation emitted by the examination object (10).

2. The multi-modal imaging system (2) according to claim 1, wherein at least parts of a detector system of the imaging system are provided in a manner integrated in the measuring head (4) as well, in particular wherein the multi-modal imaging system (2) can be battery-operated.

3. The multi-modal imaging system (2) according to one of the preceding claims, comprising at least one further system for providing overview images of the examination object (10), wherein the further system is at least partially integrated in the measuring head (4) as well, and wherein the further system comprises a CCD camera or CMOS camera (64) and/or an optical coherence tomograph, in particular wherein the further system is configured to use the near-infrared femtosecond laser radiation as illumination radiation, preferably
wherein the CCD camera or CMOS camera (64) is arranged laterally at the front of the measuring head (4) in compliance with the Scheimpflug principle, or
wherein the CCD camera or CMOS camera (64) uses the focussing optics (30) as an imaging element.

4. The multi-modal imaging system (2) according to one of the preceding claims, wherein the confocal detection device is arranged to reduce the amplitude of the central reflex, such that the confocal detection device taps a partially transmitted signal of a deflection mirror (60) arranged upstream of the radiation source (12), or
wherein a polarisation beam splitter (22) for separating linearly polarised excitation radiation and remitted unpolarised secondary radiation of the examination object (10) is provided for reducing an amplitude of the central reflex, and the confocal detection device is arranged in such a way that it receives the remitted unpolarised secondary radiation of the examination object (10).

5. The multi-modal imaging system (2) according to one of the preceding claims, wherein the confocal detection device comprises a device for time-resolved signal processing of the remission signal of the excitation beam (21) of near-infrared femtosecond laser radiation.

6. The multi-modal imaging system (2) according to one of the preceding claims, wherein the multi-modal imaging system (2) comprises a device for determining the position of a coverslip (106) and/or the examination object (10), for providing an autofocus function.

7. The multi-modal imaging system (2) according to claim 6, with an OC measurement beam (92) aligned collinearly to the optical axis of the focussing optics (30), with the aid of which the position of a coverslip (106) and/or of the examination object (10) can be determined, for providing the autofocus function.

8. The multi-modal imaging system (2) according to one of the preceding claims, comprising a pressure sensor for detecting a surface of the examination object (10) and/or for controlling a contact pressure.

9. The multi-modal imaging system (2) according to one of the preceding claims, wherein a release controller for the excitation beam (21) is coupled to a device for determining the presence of the examination object (10) in the measuring range.

10. A method for non-invasive examination of an examination object (10) using a multi-modal imaging system (2) according to any one of the preceding claims, said method comprising the following steps:
aligning the focussing optics (30) with a measurement position,
directing the near-infrared femtosecond laser radiation of the radiation source (12) at the measurement position, and
measuring the emitted secondary radiation of the examination object (10) to produce a high-resolution detailed image of the examination object (10) at the measurement position, either successively or simultaneously by the multiphoton imaging system and by the confocal detection device.

11. The method according to claim 10, comprising the following further steps:
aligning the measuring head (4) to an overview area of the examination object (10),
recording an overview image of the examination object (10),by the confocal detection system and/or by a CCD camera or CMOS camera (64) and/or an optical coherence tomograph (74), and
selecting a measurement position in the overview area to record the high-resolution detailed image.

12. The method according to claim 10 or 11, wherein axial movement artefacts of the examination object (10) are corrected by an autofocus function by continuously mechanically readjusting the distance of the focussing optics (30) to the examination object (10).

13. The method according to any one of claims 10 to 12, wherein measurement signals are evaluated with the aim of locating the surface of the examination object (10), in particular for providing an autofocus function.

14. The method according to any one of claims 10 to 13, wherein an overview image is recorded by a CCD camera or CMOS camera (64) and/or by an optical coherence tomograph (74), and wherein
when taking the overview image of the examination object (10), a distance is set between the focussing optics (30) and the examination object (10) which is set to be greater than when taking the detailed image of the examination object (10), and/or
wherein the overview image is recorded as an oblique image by the CCD camera or CMOS camera (64) and/or by the optical coherence tomography device (74) in a manner not collinear with respect to the optical axis of the focussing optics (30).

15. Use of a multi-modal imaging system (2) according to any one of claims 1 to 9 for examining living matter of the examination object (10).

## Revendications

1. Système d'imagerie multimodal (2) pour la production d'une image d'un objet à examiner (10),
avec un système d'imagerie multi-photons pour la production d'images détaillées à haute résolution de l'objet à examiner (10), qui comprend une source de rayonnement (12) avec une tête de laser (14) et une commande de laser (16), qui génère un rayonnement d'excitation (21) proche du rayonnement laser femtoseconde infrarouge, pour le déclenchement d'un rayonnement secondaire émis par l'objet à examiner (10), un système de détection (40, 44) pour une imagerie TPF, SHG et THG et/ou un système de détection pour un comptage de photons individuels corrélé dans le temps, et une optique de focalisation (30) au moyen de laquelle le rayonnement de la source de rayonnement (12) peut être orienté vers une position de mesure de l'objet à examiner (10),
dans lequel l'optique de focalisation (30) et la tête de laser (14) de la source de rayonnement (12) sont disposées dans une tête de mesure (4) qui peut pivoter, tourner et être positionnée de manière flexible librement dans l'espace, de sorte que l'examen de l'objet à examiner (10) peut être effectué sous des angles spatiaux quelconques et dans lequel la tête de laser (14) comprend une unité SHG pour le doublage de la fréquence du rayonnement émis par la commande de laser (16), afin de produire le rayon lumineux d'excitation (21) polarisé linéairement,
et avec au moins un dispositif de détection confocal qui est intégré au moins partiellement dans la tête de mesure (4) et qui est conçu pour recevoir un signal, réémis par l'objet à examiner (10), du rayon d'excitation (21) proche du rayonnement laser femtoseconde infrarouge.

2. Système d'imagerie multimodal (2) selon la revendication 1, dans lequel au moins des parties d'un système de détection du système d'imagerie sont intégrées dans la tête de mesure (4), plus particulièrement dans lequel le système d'imagerie multimodal (2) peut fonctionner avec des batteries.

3. Système d'imagerie multimodal (2) selon l'une des revendications précédentes, avec au moins un système supplémentaire pour la production de vues d'ensemble de l'objet à examiner (10), dans lequel le système supplémentaire est intégré au moins partiellement dans la tête de mesure (4) et dans lequel le système supplémentaire comprend une caméra CCD ou une caméra CMOS (64) et/ou un tomographe par cohérence optique, plus particulièrement dans lequel le système supplémentaire est conçu pour utiliser le rayonnement laser femtoseconde infrarouge en tant que rayonnement d'éclairage, de préférence
dans lequel la caméra CCD ou la caméra CMOS (64) est disposée latéralement à l'avant de la tête de mesure (4) en respectant la règle de Scheimpflug ou
dans lequel la caméra CCD ou la caméra CMOS (64) utilise l'optique de focalisation (30) en tant qu'élément de représentation.

4. Système d'imagerie multimodal (2) selon l'une des revendications précédentes, dans lequel, pour la réduction d'une amplitude du réflexe central, le dispositif de détection confocal est disposé de façon à capturer un signal partiellement transmis d'un miroir de renvoi (60) disposé en amont de la source de rayonnement (12) ou
dans lequel, pour la réduction d'une amplitude du réflexe central, un diviseur de faisceau de polarisation (22) est prévu pour la séparation du rayonnement d'excitation polarisé linéairement et du rayonnement secondaire non polarisé réémis de l'objet à examiner (10) et le dispositif de détection confocal est disposé de façon à recevoir le rayonnement secondaire non polarisé réémis de l'objet à examiner (10).

5. Système d'imagerie multimodal (2) selon l'une des revendications précédentes, dans lequel le dispositif de détection confocal comprend un dispositif de traitement, avec résolution temporelle, du signal de réémission du rayonnement d'excitation (21) proche du rayonnement laser femtoseconde infrarouge.

6. Système d'imagerie multimodal (2) selon l'une des revendications précédentes, dans lequel le système d'imagerie multimodal (2) comprend, pour la mise à disposition d'une fonction autofocus, un dispositif de détermination de la position d'un verre de protection (106) et/ou de l'objet à examiner (10).

7. Système d'imagerie multimodal (2) selon la revendication 6, avec un rayon de mesure OC (92) orienté de manière colinéaire avec l'axe optique de l'optique de focalisation (30), à l'aide duquel la position d'un verre de protection (106) et/ou de l'objet à examiner (10) peut être déterminée, afin de mettre à disposition la fonction autofocus.

8. Système d'imagerie multimodal (2) selon l'une des revendications précédentes, avec un capteur de pression pour la détection d'une surface de l'objet à examiner (10) et/ou pour le contrôle d'une pression.

9. Système d'imagerie multimodal (2) selon l'une des revendications précédentes, dans lequel une commande de libération du rayonnement d'excitation (21) est couplée à un dispositif pour la détection de la présence de l'objet à examiner (10) dans l'intervalle de mesure.

10. Procédé d'examen non invasif d'un objet à examiner (10) à l'aide d'un système d'imagerie multimodal (2) selon l'une des revendications précédentes, avec les étapes suivantes :
orientation de l'optique de focalisation (30) vers une position de mesure,
orientation du rayonnement laser femtoseconde infrarouge de la source de rayonnement (12) vers la position de mesure et
mesure du rayonnement secondaire émis de l'objet à examiner (10) afin de créer une image détaillée à haute résolution de l'objet à examiner (10) au niveau de la position de mesure successivement ou simultanément par le système d'imagerie multi-photons et par le dispositif de détection confocal.

11. Procédé selon la revendication 10, avec les étapes supplémentaires suivantes :
orientation de la tête de mesure (4) vers une zone de vue d'ensemble de l'objet à examiner (10),
enregistrement d'une vue d'ensemble de l'objet à examiner (10) par le système de détection confocal et/ou par une caméra CCD ou une caméra CMOS (64) et/ou un tomographe par cohérence optique (74) et
sélection d'une position de mesure dans la zone de vue d'ensemble pour l'enregistrement de l'image détaillée à haute résolution.

12. Procédé selon la revendication 10 ou 11, dans lequel des artefacts de déplacement axiaux de l'objet à examiner (10) sont corrigés par une fonction autofocus, en effectuant un ajustement mécanique ultérieur de la distance entre l'optique de focalisation (30) et l'objet à examiner (10).

13. Procédé selon l'une des revendications 10 à 12, dans lequel les signaux de mesure sont analysés dans le but de détecter la surface de l'objet à examiner (10), plus particulièrement pour la mise à disposition d'une fonction autofocus.

14. Procédé selon l'une des revendications 10 à 13, dans lequel une vue d'enseigner est enregistrée par une caméra CCD ou une caméra CMOS (64) et/ou par un tomographe par cohérence optique (74) et dans lequel
lors de l'enregistrement de la vue d'ensemble de l'objet à examiner (10), une distance, ajustée entre l'optique de focalisation (30) et l'objet à examiner (10) est plus grande que lors de l'enregistrement de l'image détaillée de l'objet à examiner (10) et/ou
dans lequel la vue d'ensemble provenant de la caméra CCD ou de la caméra CMOS (64) et/ou du tomographe par cohérence optique (74) est enregistrée sous la forme d'une image inclinée non colinéaire par rapport à l'axe optique de l'optique de focalisation (30).

15. Utilisation d'un système d'imagerie multimodal (2) selon l'une des revendications 1 à 9 pour l'examen d'une matière vivante de l'objet à examiner (10).
